# EUROPEAN PATENT APPLICATION

(11) **EP 4 215 214 A1**
(43) Date of publication of application: **26.07.2023**
(21) Application number: 21869389.3
(22) Date of filing: 15.09.2021
(51) Int. Cl.: A61K 48/00, A61K 38/02, A61K 39/395, A61K 45/00, A61P 35/00, A61P 35/02, A61P 37/04, A61P 43/00, C12Q 1/68, G01N 33/15, G01N 33/50, C12N 15/12

(54) **CANCER THERAPEUTIC AGENT, IMMUNOSTIMULANT AND SCREENING METHOD FOR ANTICANCER SUBSTANCE**

(30) Priority: 16.09.2020 JP 2020155901
(71) Applicant: OSAKA UNIVERSITY, Suita-shi Osaka 565-0871 (JP)
(72) Inventor: NIMURA Keisuke, Suita-shi, Osaka 565-0871 (JP); ISHIBASHI Airi, Suita-shi, Osaka 565-0871 (JP)
(74) Representative: Witte, Weller & Partner Patentanwälte mbB
(86) International application number: PCT/JP2021/033925
(87) International publication number: WO 2022/059703

(57) **Abstract**

The present invention provides a medicament for treating cancer comprising, as an active ingredient, an apolipoprotein and/or interferon regulatory factor 7 (IRF7), wherein the expression of the apolipoprotein and IRF7 is increased in cancer cells by treatment of the cancer cells with hemagglutinating virus of Japan-envelope (HVJ-E); the medicament for treating cancer used in combination with a T-cell co-stimulator agonist; and an immunostimulant comprising a combination of a T-cell co-stimulator agonist and at least one selected from the group consisting of an apolipoprotein, IRF7 and HVJ-E.

## Description

### TECHNICAL FIELD

The present invention relates to a medicament for treating cancer, an immunostimulant, and a method for screening for an anti-cancer substance.

### BACKGROUND ART

Immunotherapy in cancer treatment has recently attracted much attention thanks to the development of immune checkpoint inhibitor therapy and CAR-T cell therapy. The basic concept of this approach is to maintain the anti-cancer activity and number of killer T cells. However, many patients are nonresponsive to immune checkpoint inhibitor therapy in practical setting. CAR-T cell therapy has limitations in that it is dependent on the specificity of the antibody used and typically targets only a single tumor antigen, although cancers are characterized by a variety of neoantigen combinations and intratumoral heterogeneity. Therefore, enhanced induction of anti-tumor immunity in the body would be effective in cancer treatment, but such a therapeutic approach has not yet been developed.

Oncolytic virotherapy medicine is attracting attention as a novel agent for tumor treatment and is exemplified by T-VEC, which is a herpes simplex virus (HSV) loaded with GM-CSF, a cytokine that activates dendritic cells. T-VEC has been approved by the FDA as a medicine for the treatment of melanoma. T-VEC has an oncolytic activity and a potential for inducing systemic anti-tumor effect through GM-CSF-mediated enhancement of antigen-presenting ability, but in reality, its anti-tumor effect on non-target tumors is limited.

Hemagglutinating virus of Japan-envelope (HVJ-E) is a completely inactivated and purified hemagglutinating virus of Japan (HVJ) particle that only retains membrane fusion activity of the outer membrane. Unlike conventional oncolytic viruses, HVJ-E is non-proliferative and thus can safely activate the immune system, thereby inducing anti-tumor effect (Non-Patent Literature 1). In a phase I/IIa clinical trial of HVJ-E therapy in patients with stage IIIC and IV melanoma, anti-tumor responses were observed in 3 of 6 target tumors (Non-Patent Literature 2). In the phase I clinical trial, intratumoral injection of HVJ-E had a remarkable anti-tumor effect, with partial responses (PRs) observed in 66.6% of patients as assessed by RECIST, which includes assessment of tumor size in treated or non-treated lesions. In a phase I clinical trial in patients with relapsed castration-resistant prostate cancer or malignant pleural mesothelioma, 5 of 6 prostate cancer patients treated with high-dose HVJ-E had stable disease (SD) (see Non-Patent Literature 3), and 3 of 3 malignant pleural mesothelioma patients treated with HVJ-E had SD. This proves that HVJ-E has high anti-tumor efficacy in actual cancer patients.

Oncolytic virotherapy is a promising cancer therapy based on a new mechanism, but the molecular mechanism underlying such high anti-tumor effect is still poorly understood. In addition, there are various problems with oncolytic virotherapy, such as big obstacles for using viruses as medicines in ordinary hospitals, difficulties in large and stable supply of viruses as medicines, and impracticality of frequent administration because of unwanted antibody production against viruses.

HVJ-E therapy, which uses a non-proliferative virus, is safer than other oncolytic virotherapies, but antibody production against the virus itself is unavoidable like other oncolytic virotherapies. In addition, HVJ-E therapy has a risk of thrombosis due to the hemagglutination ability of HVJ. Furthermore, HVJ-E is effective in tumor suppression in directly treated lesions, but its tumor suppressive effect in non-treated lesions (distant lesions) is limited.

ApoA1 has anti-tumor effects, which are disclosed in Patent Literature 1 and Non-Patent Literature 4. However, these effects are supported by the data that administration of a large amount of ApoA1 collected from human plasma to ApoA1-deficient transgenic mice resulted in inhibition of cancer cell engraftment. That is, no confirmation has been made of anti-tumor effects of ApoA1 on tumors in wild-type mice, in which ApoA1 is naturally present in the plasma.

### CITATION LIST

### Patent Literature

Patent Literature 1: US 2011/0305707 A1

### Non-Patent Literature

Non-Patent Literature 1:
   Kurooka et al., Cancer Res January 1 2007 67 (1) 227-236
Non-Patent Literature 2:
   Kiyohara et al., Cancer Immunology, Immunotherapy 2020 69, 1131-1140
Non-Patent Literature 3:
   Fujita et al., Cancer science 2020 111: 1692-1698
Non-Patent Literature 4:
   Zamanian-Daryoush et al., J Biol Chem. 2013 288(29):21237-21252

### SUMMARY OF INVENTION

### TECHNICAL PROBLEM

Objects of the present invention are to elucidate the molecular mechanism of the anti-tumor effect of HVJ-E, to provide a medicament for treating cancer that is capable of providing an anti-tumor effect equivalent to that of HVJ-E without the use of HVJ-E, and to provide a medicament for treating cancer that is capable of providing an anti-tumor effect on cancer tissue located at a distance from treated target cancer tissue. Another object of the present invention is to provide an immunostimulant that is capable of stimulating immunity in distant lesions, thereby providing therapeutic effects on diseases other than cancer. Yet another object of the present invention is to provide a method for screening for an anti-cancer substance that is capable of providing an anti-tumor effect equivalent to that of HVJ-E without the use of HVJ-E.

### SOLUTION TO PROBLEM

The present invention includes the following to achieve the above-mentioned objects.
[1] A medicament for treating cancer comprising, as an active ingredient, a gene product whose expression is increased in cancer cells by treatment of the cancer cells with hemagglutinating virus of Japan-envelope.
[2] The medicament for treating cancer according to the above [1], wherein the active ingredient is an apolipoprotein and/or interferon regulatory factor 7.
[3] The medicament for treating cancer according to the above [1] or [2], wherein the medicament is used in combination with a T-cell co-stimulator agonist.
[4] A medicament for treating cancer comprising a combination of hemagglutinating virus of Japan-envelope and a T-cell co-stimulator agonist.
[5] The medicament for treating cancer according to the above [3] or [4], wherein the medicament is used in combination with a CD4-positive T-cell depleting antibody.
[6] An immunostimulant comprising a combination of a T-cell co-stimulator agonist and at least one selected from the group consisting of an apolipoprotein, interferon regulatory factor 7, and hemagglutinating virus of Japan-envelope.
[7] The immunostimulant according to the above [6], wherein the immunostimulant is for local administration.
[8] The immunostimulant according to the above [6] or [7], wherein the immunostimulant is used in combination with a CD4-positive T-cell depleting antibody.
[9] A method for screening for an anti-cancer substance, comprising the steps of:
   (1) bringing cancer cells into contact with a test substance,
   (2) measuring the expression level of interferon regulatory factor 7 (IRF7) in the cancer cells, and
   (3) comparing the expression level of IRF7 measured in the previous step to that in the absence of contact with the test substance to identify a substance capable of increasing the expression level of IRF7.
[10] A method for screening for an anti-cancer substance, comprising the steps of:
   (1) bringing cancer cells into contact with a test substance,
   (2) measuring the expression level of an apolipoprotein in the cancer cells, and
   (3) comparing the expression level of the apolipoprotein measured in the previous step to that in the absence of contact with the test substance to identify a substance capable of increasing the expression level of the apolipoprotein.

### ADVANTAGEOUS EFFECTS OF INVENTION

The present invention provides a medicament for treating cancer that is capable of providing an anti-tumor effect equivalent to that of HVJ-E; and a medicament for treating cancer that is capable of providing an anti-tumor effect on non-target cancer tissue, which is located at a distance from target cancer tissue directly treated with the medicament and is not subjected to direct treatment with the medicament. In addition, the present invention provides an immunostimulant that is capable of promoting migration of migrated T cells from local lesions to distant lesions. Furthermore, the screening method of the present invention can be used to screen for an anti-cancer substance that is capable of providing an anti-tumor effect equivalent to that of HVJ-E.

### BRIEF DESCRIPTION OF DRAWINGS

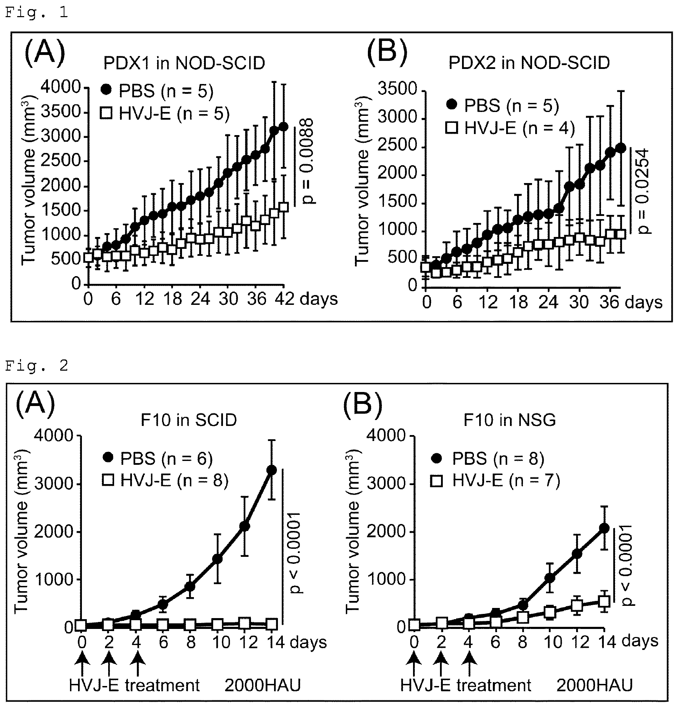

Fig. 1 shows the tumor volumes recorded in NOD-SCID mice subjected to transplantation of tumor tissue grafts from melanoma patients and subsequent intratumoral treatment with HVJ-E or PBS. Fig. 1A shows the results obtained in mice bearing a tumor tissue graft transplanted from a patient in the first case, and Fig. 1B shows the results obtained in mice bearing a tumor tissue graft transplanted from a patient in the second case.
Fig. 2 shows the tumor volumes recorded in SCID or NSG mice subjected to transplantation of B16F10 cells (mouse melanoma cells) and subsequent intratumoral treatment with HVJ-E or PBS. Fig. 2A shows the results obtained in SCID mice, and Fig. 2B shows the results obtained in NSG mice.

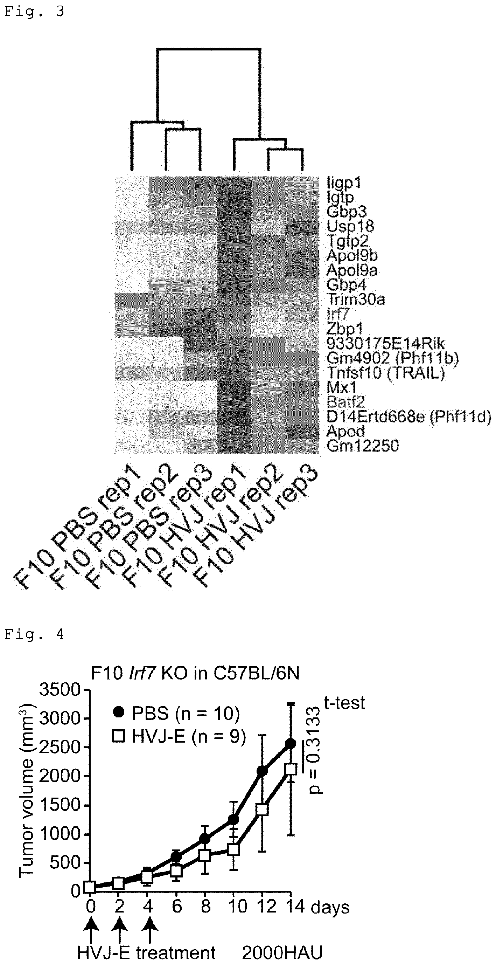

Fig. 3 shows the results of analysis for genes whose expression is increased by HVJ-E treatment in tumors harvested from GFP mice subjected to transplantation of B16F10 cells and subsequent intratumoral treatment with HVJ-E or PBS.
Fig. 4 shows the tumor volumes recorded in C57BL/6N mice subjected to transplantation of Irf7-deficient B16F10 cells and subsequent intratumoral treatment with HVJ-E or PBS.

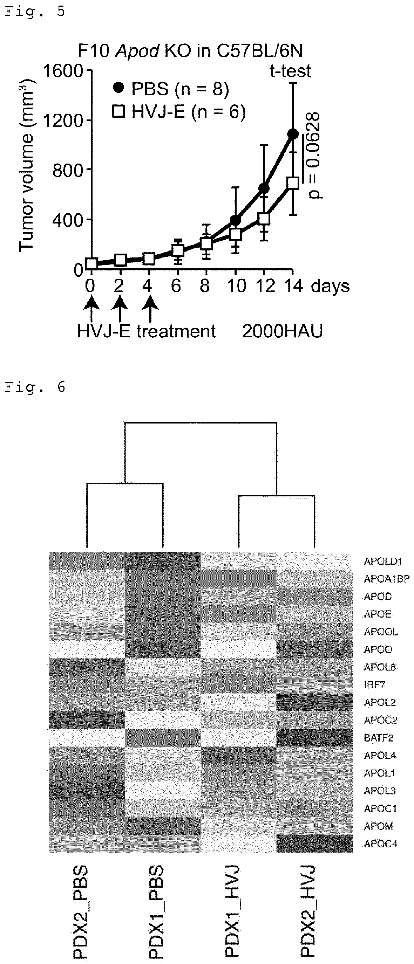

Fig. 5 shows the tumor volumes recorded in C57BL/6N mice subjected to transplantation of Apod-deficient B16F10 cells and subsequent intratumoral treatment with HVJ-E or PBS.
Fig. 6 shows the results of analysis for genes whose expression is increased by HVJ-E treatment in tumors harvested from NOD-SCID mice subjected to transplantation of tumor tissue grafts from melanoma patients and subsequent intratumoral treatment with HVJ-E or PBS (the tumor volumes recorded in the mice are shown in Fig. 1).

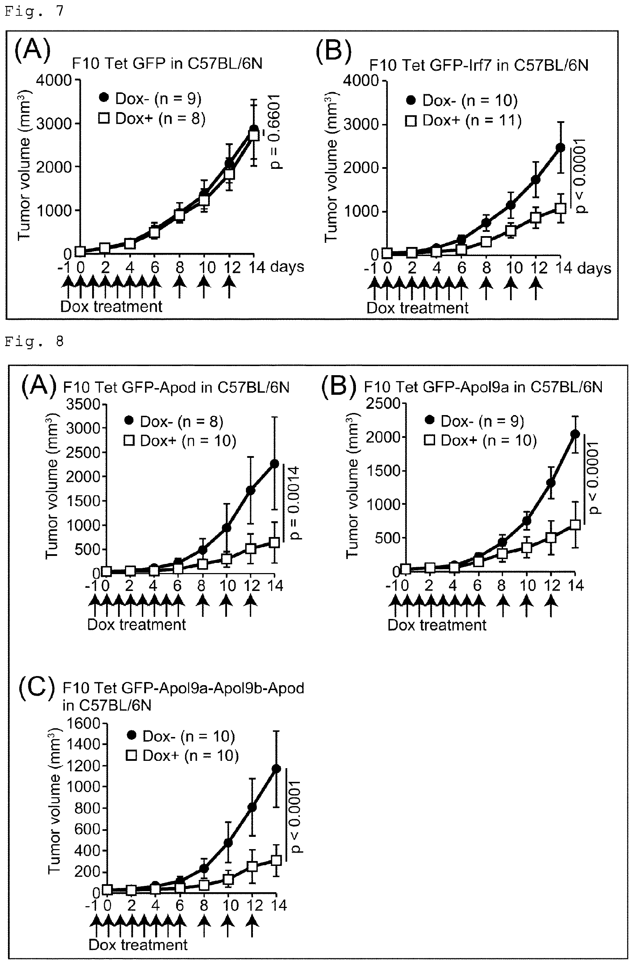

Fig. 7 shows the tumor volumes recorded in C57BL/6N mice subjected to transplantation of Tet GFP-Irf7- or Tet GFP-transfected B16F10 cells and subsequent treatment with or without of doxycycline (Dox). Fig. 7A shows the results obtained in the Tet GFP group, and Fig. 7B shows the results obtained in the Tet GFP-Irf7 group.
Fig. 8 shows the tumor volumes recorded in C57BL/6N mice subjected to transplantation of Tet GFP-Apod-, Tet GFP-Apol9a-, or Tet GFP-Apol9a-Apol9b-Apod-transplanted B16F10 cells and subsequent treatment with or without of doxycycline (Dox). Fig. 8A shows the results obtained in the Tet GFP-Apod group, Fig. 8B shows the results obtained in the Tet GFP-Apol9a group, and Fig. 8C shows the results obtained in the Tet GFP-Apol9a-Apol9b-Apod group.

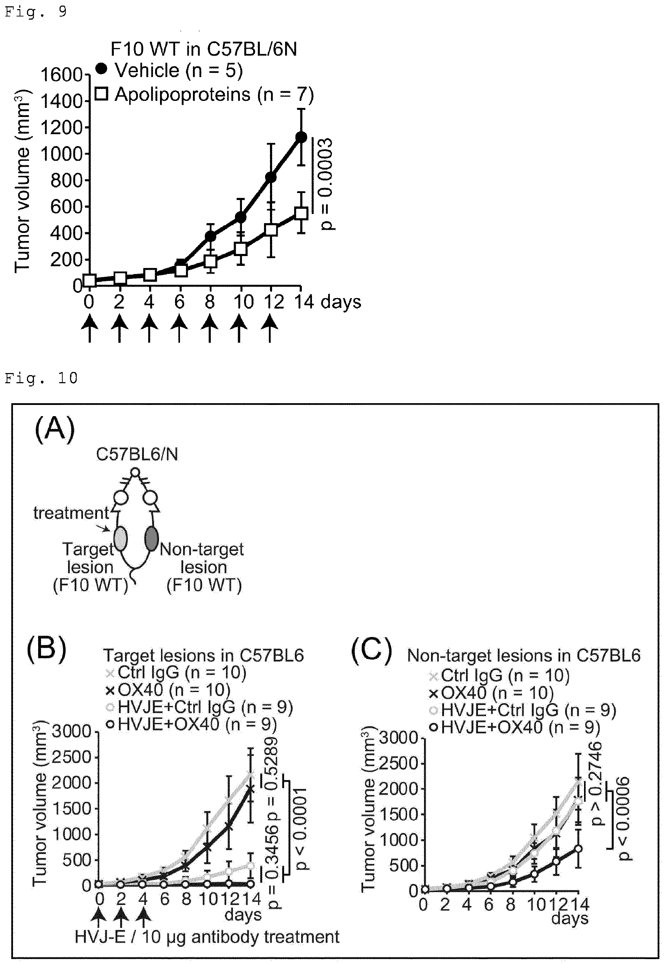

Fig. 9 shows the tumor volumes recorded in C57BL/6N mice subjected to transplantation of B16F10 cells (wild type) and subsequent intratumoral treatment with a mixture of three recombinant apolipoproteins D, L9a and L9b, or a vehicle.
Fig. 10 shows the tumor volumes of target (left) and non-target (right) tumors recorded in C57BL/6N mice subjected to bilateral transplantation of B16F10 cells (wild type) and subsequent treatment of the left tumor with HVJ-E and OX40 agonistic antibody (HVJ-E + OX40 group), HVJ-E and control IgG (HVJ-E + Ctrl IgG group), OX40 agonistic antibody alone (OX40 group), or control IgG alone (Ctrl IgG group). Fig. 10A shows a schematic representation of a tumor-bearing mouse, Fig. 10B shows the results obtained in the target tumor lesion, and Fig. 10C shows the results obtained in the non-target tumor lesion.

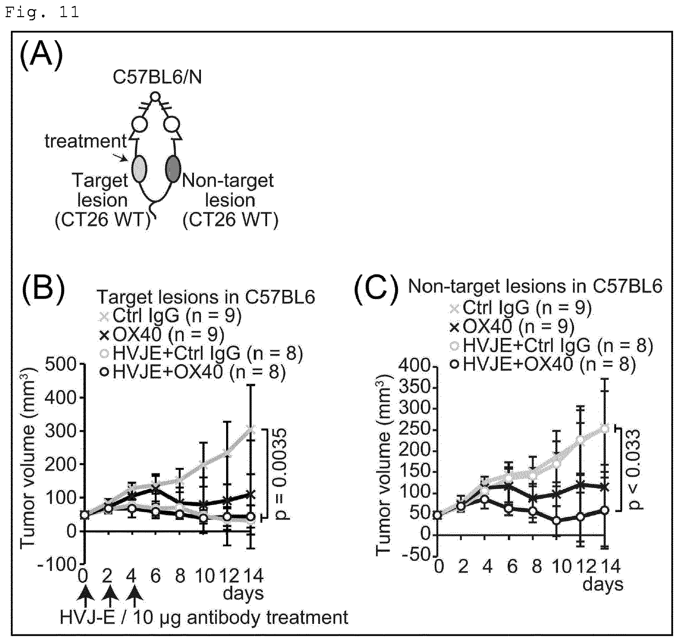

Fig. 11 shows the tumor volumes of target (left) and non-target (right) tumors recorded in C57BL/6N mice subjected to bilateral transplantation of CT26 cells (wild type) and subsequent treatment of the left tumor with HVJ-E and OX40 agonistic antibody (HVJ-E + OX40 group), HVJ-E and control IgG (HVJ-E + Ctrl IgG group), OX40 agonistic antibody alone (OX40 group), or control IgG alone (Ctrl IgG group). Fig. 11A shows a schematic representation of a tumor-bearing mouse, Fig. 11B shows the results obtained in the target tumor lesion, and Fig. 11C shows the results obtained in the non-target tumor lesion.

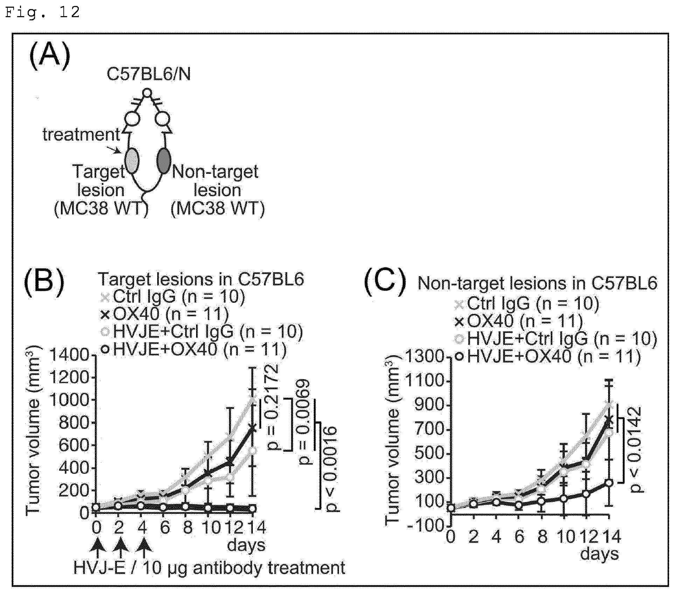

Fig. 12 shows the tumor volumes of target (left) and non-target (right) tumors recorded in C57BL/6N mice subjected to bilateral transplantation of MC38 cells (wild type) and subsequent treatment of the left tumor with HVJ-E and OX40 agonistic antibody (HVJ-E + OX40 group), HVJ-E and control IgG (HVJ-E + Ctrl IgG group), OX40 agonistic antibody alone (OX40 group), or control IgG alone (Ctrl IgG group). Fig. 12A shows a schematic representation of a tumor-bearing mouse, Fig. 12B shows the results obtained in the target tumor lesion, and Fig. 12C shows the results obtained in the non-target tumor lesion.

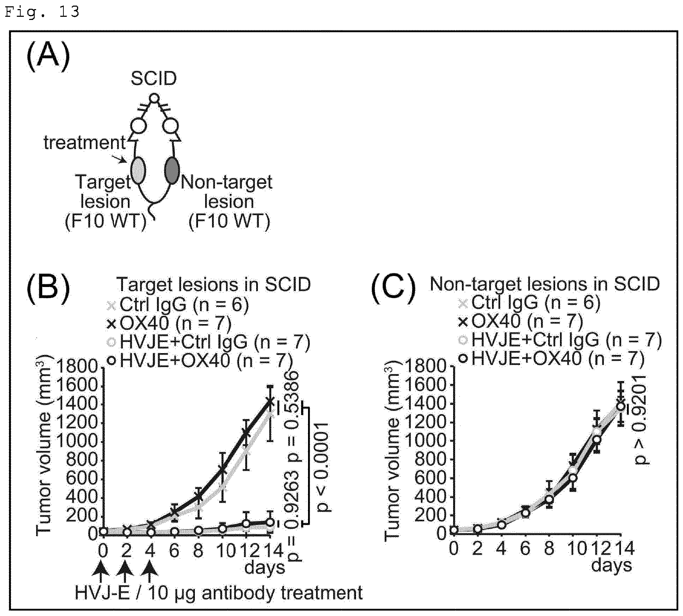

Fig. 13 shows the tumor volumes of target (left) and non-target (right) tumors recorded in SCID mice subjected to bilateral transplantation ofB16F10 cells (wild type) and subsequent treatment of the left tumor with HVJ-E and OX40 agonistic antibody (HVJ-E + OX40 group), HVJ-E and control IgG (HVJ-E + Ctrl IgG group), OX40 agonistic antibody alone (OX40 group), or control IgG alone (Ctrl IgG group). Fig. 13A shows a schematic representation of a tumor-bearing mouse, Fig. 13B shows the results obtained in the target tumor lesion, and Fig. 13C shows the results obtained in the non-target tumor lesion.

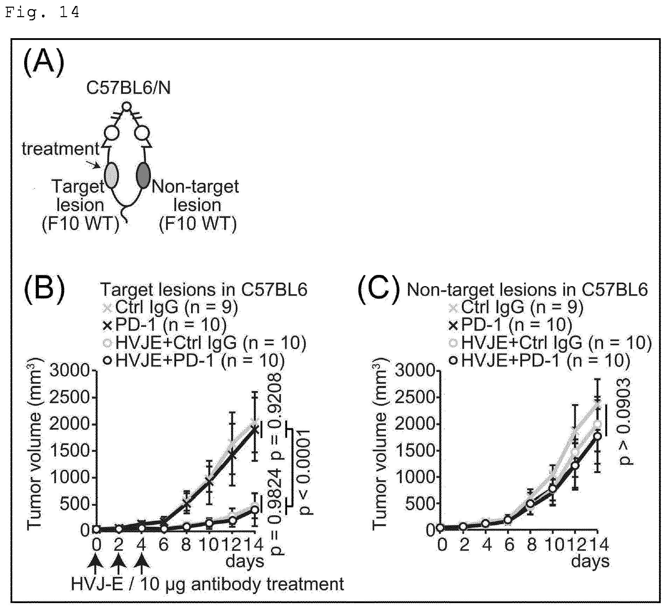

Fig. 14 shows the tumor volumes of target (left) and non-target (right) tumors recorded in C57BL/6N mice subjected to bilateral transplantation of B16F10 cells (wild type) and subsequent treatment of the left tumor with HVJ-E and anti PD-1 antibody, which is an immune checkpoint inhibitor (HVJ-E + PD-1 group), HVJ-E and control IgG (HVJ-E + Ctrl IgG group), anti-PD-1 antibody alone (PD-1 group), or control IgG alone (Ctrl IgG group). Fig. 14A shows a schematic representation of a tumor-bearing mouse, Fig. 14B shows the results obtained in the target tumor lesion, and Fig. 14C shows the results obtained in the non-target tumor lesion.

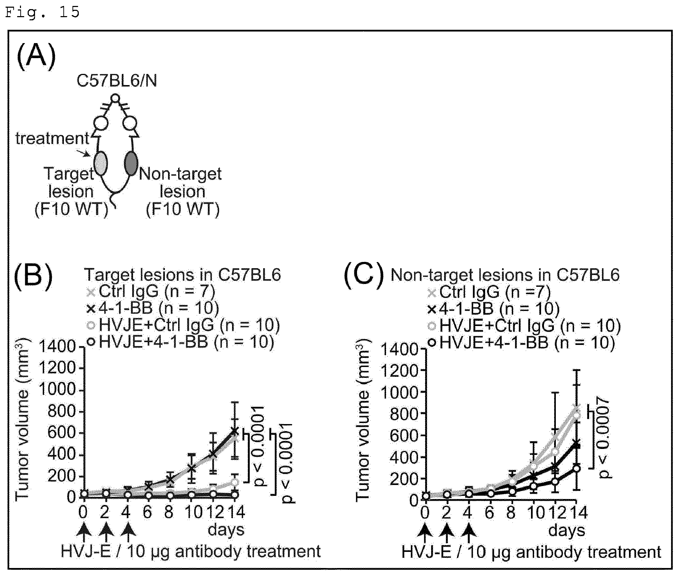

Fig. 15 shows the tumor volumes of target (left) and non-target (right) tumors recorded in C57BL/6N mice subjected to bilateral transplantation of B16F10 cells (wild type) and subsequent treatment of the left tumor with HVJ-E and 4-1-BB agonistic antibody (HVJ-E + 4-1-BB group), HVJ-E and control IgG (HVJ-E + Ctrl IgG group), 4-1-BB agonistic antibody alone (4-1-BB group), or control IgG alone (Ctrl IgG group). Fig. 15A shows a schematic representation of a tumor-bearing mouse, Fig. 15B shows the results obtained in the target tumor lesion, and Fig. 15C shows the results obtained in the non-target tumor lesion.

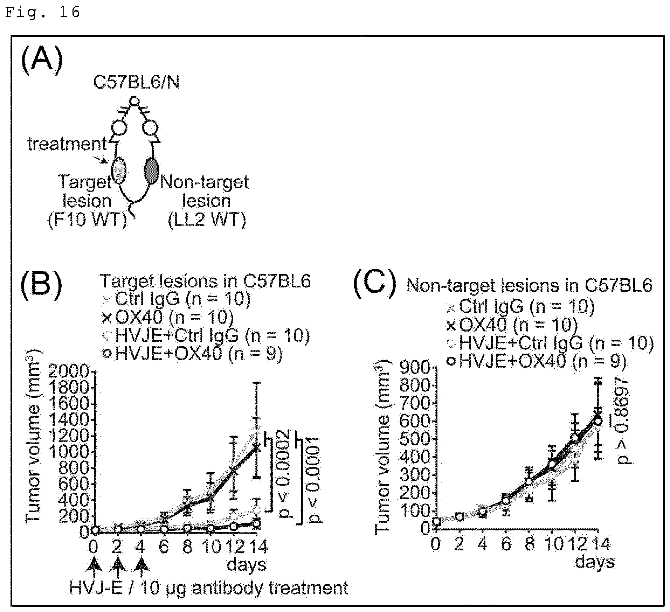

Fig. 16 shows the tumor volumes of target (left) and non-target (right) tumors recorded in C57BL/6N mice subjected to transplantation of B16F10 cells (wild type) on the left side and mouse lung adenocarcinoma LL/2 cells on the right side and subsequent treatment of the left tumor with HVJ-E and OX40 agonistic antibody (HVJ-E + OX40 group), HVJ-E and control IgG (HVJ-E + Ctrl IgG group), OX40 agonistic antibody alone (OX40 group), or control IgG alone (Ctrl IgG group). Fig. 16A shows a schematic representation of a tumor-bearing mouse, Fig. 16B shows the results obtained in the target tumor lesion, and Fig. 16C shows the results obtained in the non-target tumor lesion.

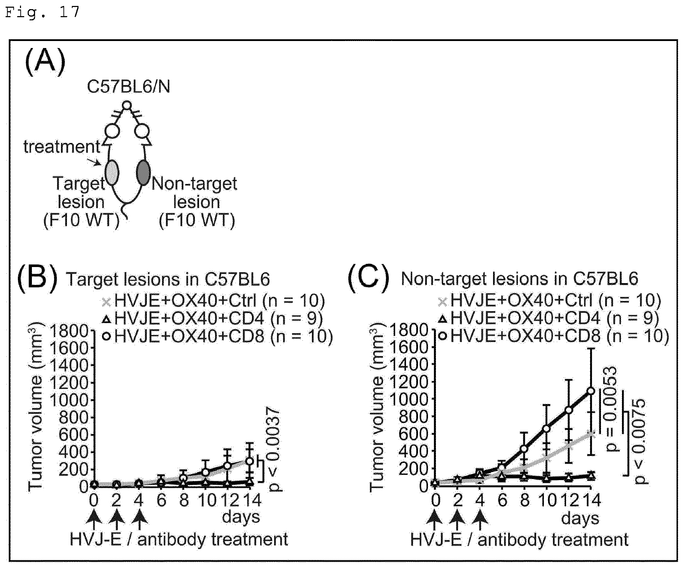

Fig. 17 shows the tumor volumes of target (left) and non-target (right) tumors recorded in C57BL/6N mice subjected to bilateral transplantation ofB16F10 cells (wild type) and subsequent treatment of the left tumor with HVJ-E and OX40 agonistic antibody plus intraperitoneal treatment with a CD4-positive T-cell depleting antibody (HVJ-E + OX40 + CD4 group), a CD8-positive T-cell depleting antibody (HVJ-E + OX40 + CD8 group), or control IgG (HVJ-E + OX40 + Ctrl group). Fig. 17A shows a schematic representation of a tumor-bearing mouse, Fig. 17B shows the results obtained in the target tumor lesion, and Fig. 17C shows the results obtained in the non-target tumor lesion.

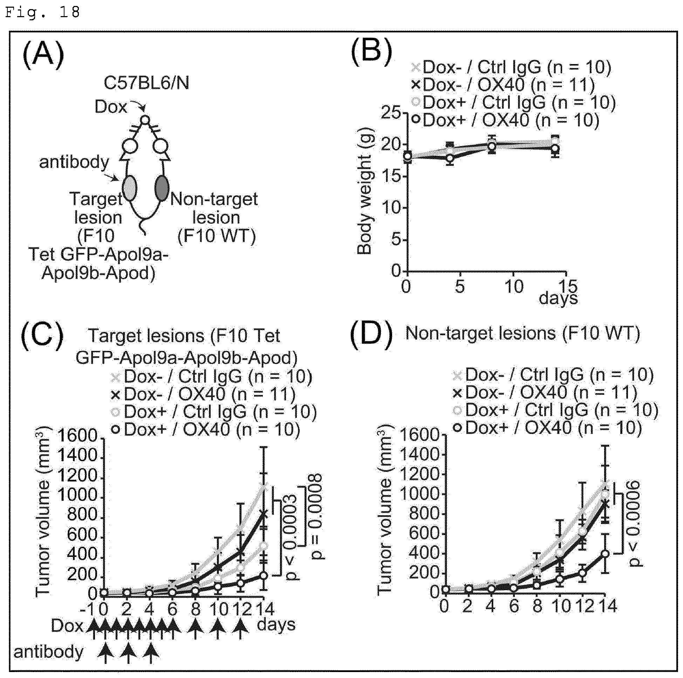

Fig. 18 shows the tumor volumes of target (left) and non-target (right) tumors recorded in C57BL/6N mice subjected to transplantation of Tet GFP-Apol9a-Apol9b-Apod-transfected B16F10 cells on the left side and B16F10 cells (wild type) on the right side and subsequent treatment of the left tumor with OX40 agonistic antibody or control IgG plus treatment with doxycycline (Dox). Fig. 18A shows a schematic representation of a tumor-bearing mouse, Fig. 18B shows the results of body weight measurement, Fig. 18C shows the results obtained in the target tumor lesion, and Fig. 18D shows the results obtained in the non-target tumor lesion.

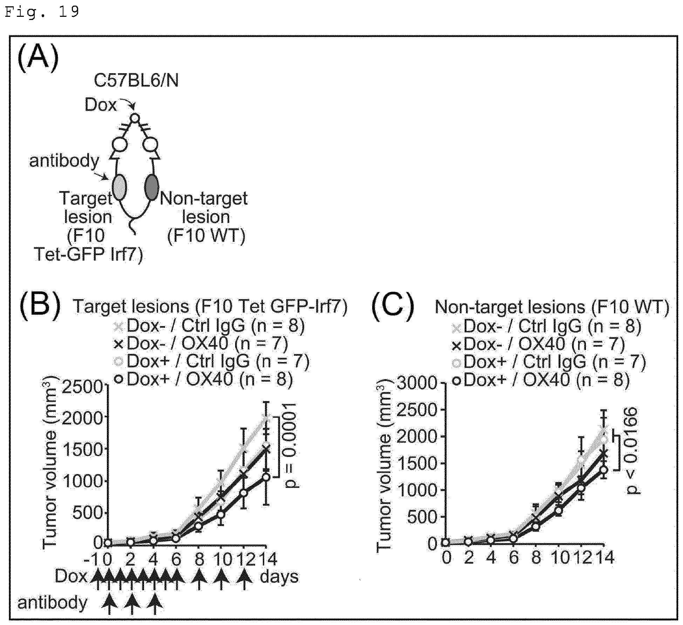

Fig. 19 shows the tumor volumes of target (left) and non-target (right) tumors recorded in C57BL/6N mice subjected to transplantation of Tet GFP-Irf7-transfected B16F10 cells on the left side and B16F10 cells (wild type) on the right side and subsequent treatment of the left tumor with OX40 agonistic antibody or control IgG plus treatment with doxycycline (Dox). Fig. 19A shows a schematic representation of a tumor-bearing mouse, Fig. 19B shows the results obtained in the target tumor lesion, and Fig. 19C shows the results obtained in the non-target tumor lesion.

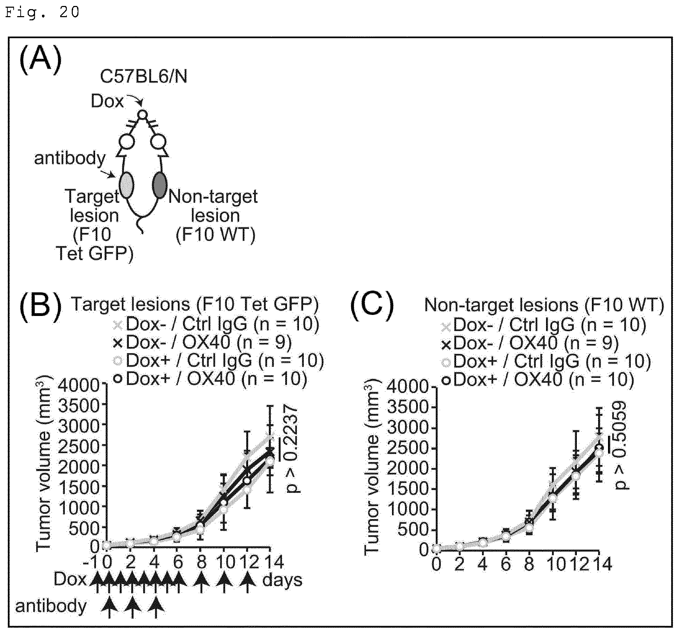

Fig. 20 shows the tumor volumes of target (left) and non-target (right) tumors recorded in C57BL/6N mice subjected to transplantation of Tet GFP-transfected B16F10 cells on the left side and B16F10 cells (wild type) on the right side and subsequent treatment of the left tumor with OX40 agonistic antibody or control IgG plus treatment with doxycycline (Dox). Fig. 20A shows a schematic representation of a tumor-bearing mouse, Fig. 20B shows the results obtained in the target tumor lesion, and Fig. 20C shows the results obtained in the non-target tumor lesion.

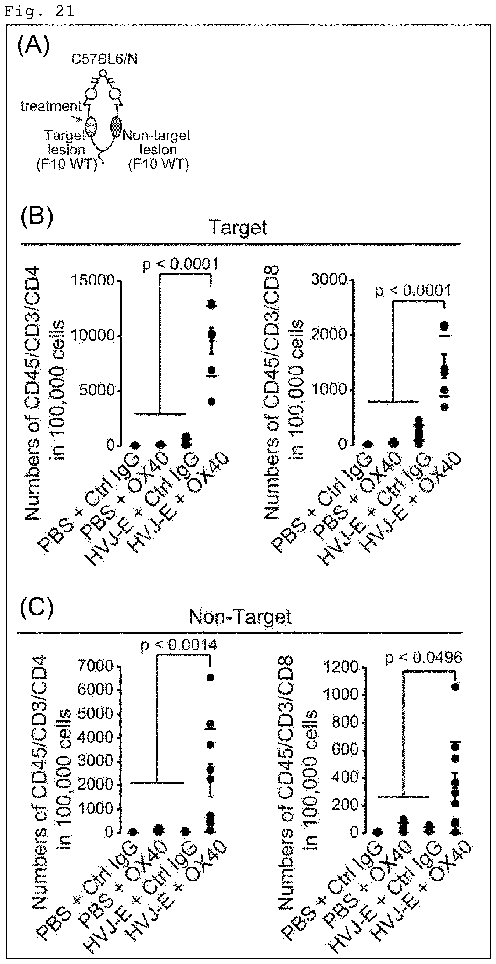

Fig. 21 shows the numbers of CD4-positive T cells and CD8-positive T cells in target and non-target tumors of C57BL/6N mice subjected to bilateral transplantation of B16F10 cells (wild type) and subsequent treatment of the left tumor with HVJ-E and OX40 agonistic antibody (HVJ-E + OX40 group), HVJ-E and control IgG (HVJ-E + Ctrl IgG group), PBS and OX40 agonistic antibody (PBS + OX40 group), or PBS and control IgG (PBS + Ctrl IgG group).

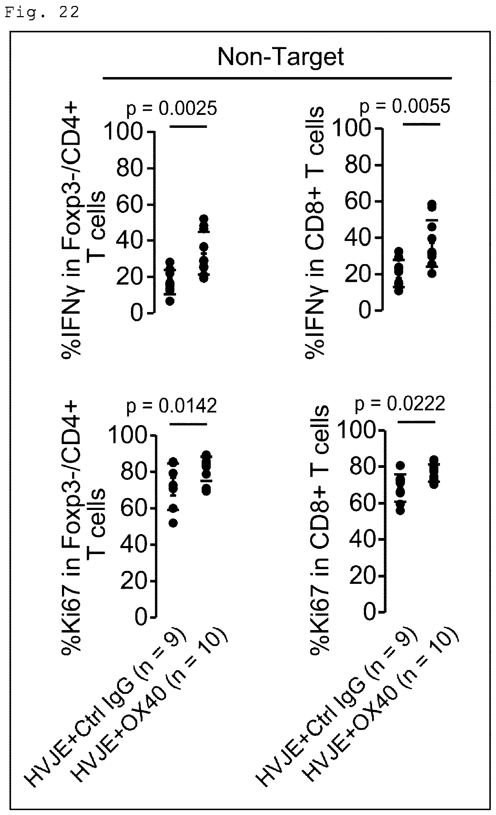

Fig. 22 shows the expression levels of IFNγ and Ki67 in the CD4-positive T cell population and in the CD8-positive T cell population in the non-target tumors in the HVJ-E + OX40 group and in HVJ-E + Ctrl IgG group in Fig. 21.

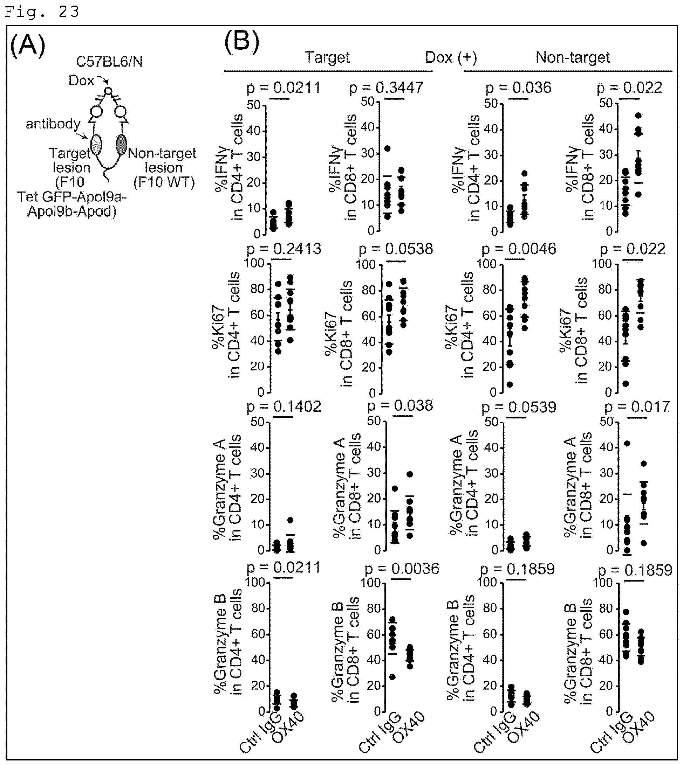

Fig. 23 shows the expression levels of IFNγ, Ki67, and granzymes A and B in the CD4-positive T cell population and in the CD8-positive T cell population in target and non-target tumors of C57BL/6N mice subjected to transplantation of Tet GFP-Apol9a-Apol9b-Apod-transfected B16F10 cells on the left side and B16F10 cells (wild type) on the right side and subsequent treatment of the left tumor with OX40 agonistic antibody or control IgG plus treatment with doxycycline (Dox).

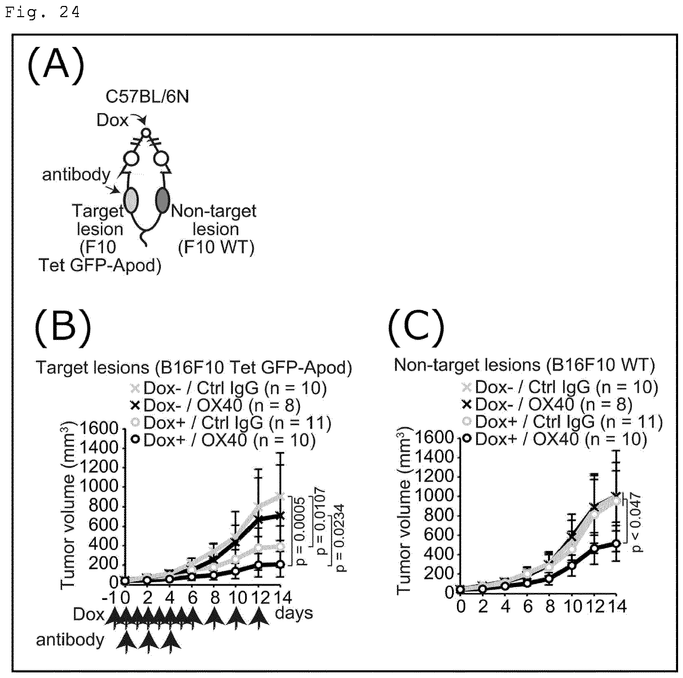

Fig. 24 shows the tumor volumes of target (left) and non-target (right) tumors recorded in C57BL/6N mice subjected to transplantation of Tet GFP-Apod-transfected B16F10 cells on the left side and B16F10 cells (wild type) on the right side and subsequent treatment of the left tumor with OX40 agonistic antibody or control IgG plus treatment with doxycycline (Dox). Fig. 24A shows a schematic representation of a tumor-bearing mouse, Fig. 24B shows the results obtained in the target tumor lesion, and Fig. 24C shows the results obtained in the non-target tumor lesion.

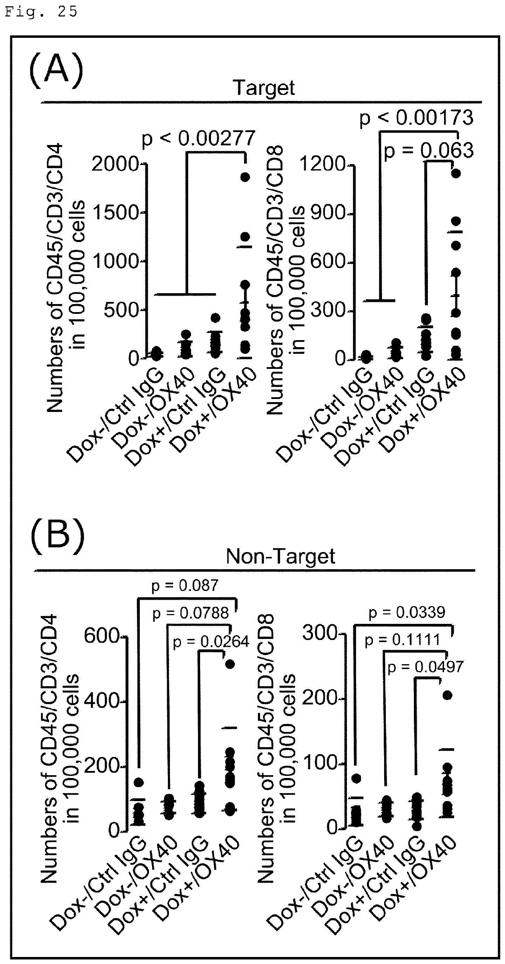

Fig. 25 shows the numbers of CD4-positive T cells and CD8-positive T cells in the target and non-target tumor lesions of the mice in Fig. 24.

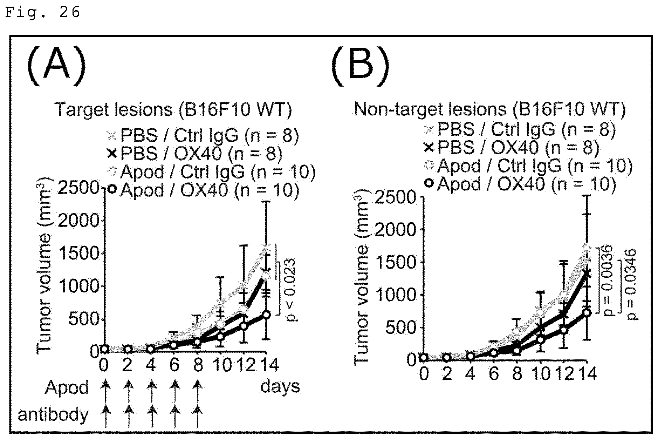

Fig. 26 shows the tumor volumes of target (left) and non-target (right) tumors recorded in C57BL/6N mice subjected to bilateral transplantation of B16F10 cells (wild type) and subsequent treatment of the left tumor with Apod and OX40 agonistic antibody (Apod/OX40 group), Apod and control IgG (Apod/Ctrl IgG group), PBS and OX40 agonistic antibody (PBS/OX40 group), or PBS and control IgG (PBS/Ctrl IgG group). Fig. 26A shows the results obtained in the target tumor lesion, and Fig. 26B shows the results obtained in the non-target tumor lesion.

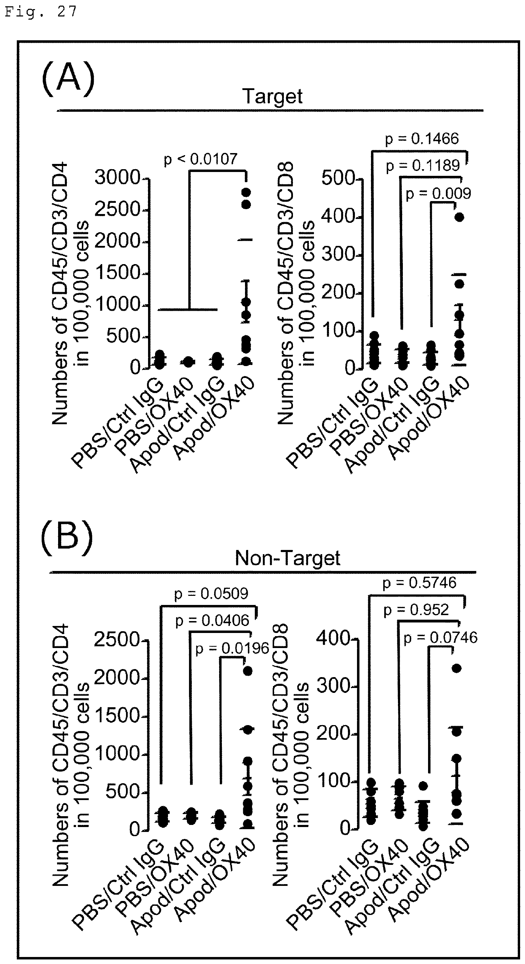

Fig. 27 shows the numbers of CD4-positive T cells and CD8-positive T cells in the target and non-target tumor lesions of the mice in Fig. 26.

### DESCRIPTION OF EMBODIMENTS

### Medicament for treating cancer

The present invention provides a medicament for treating cancer comprising, as an active ingredient, a gene product whose expression is increased in cancer cells by treatment of the cancer cells with HVJ-E. The gene product whose expression is increased in cancer cells by treatment of the cancer cells with HVJ-E can be identified using a known method. For example, tumor-bearing mice are generated, RNAs are extracted from tumors treated with or without HVJ-E, and RNA sequencing and expression comparison are performed to detect the gene product of interest. In the medicament for treating cancer, an apolipoprotein and interferon regulatory factor 7 can be used as preferable gene products whose expression is increased in cancer cells by treatment of the cancer cells with HVJ-E.

The apolipoprotein used as an active ingredient in the medicament of the present invention may be an apolipoprotein of any organism, but mammalian apolipoproteins are preferred. Mammals include, but are not limited to, humans, mice, rats, cattle, horses, pigs, dogs, and cats. Particularly preferably, human apolipoproteins are used. Examples of the human apolipoprotein include, but are not limited to, APOA1BP (also known as NAXE), APOOL, APOO, APOD, APOE, APOL1, APOL2, APOL3, APOL4, APOL5, APOL6, APOC1, APOC2, and APOC4. Preferred are APOD, APOL1, and APOL6. A single apolipoprotein or a combination of two or more apolipoproteins may be used as an active ingredient. The nucleotide and amino acid sequences of the genes encoding human apolipoproteins can be obtained from known databases such as NCBI. The amino acid and nucleotide sequences listed in Table 1 are non-limiting examples.

**[Table 1]**

| Human apolipoprotein | Amino acid sequence (RefSeq ID) | Nucleotide sequence (RefSeq ID) |
|---|---|---|
| APOA1BP (NAXE) | NP_658985.2 | NM_144772.3 |
| APOOL | NP_940852.3 | NM_198450.6 |
| APOO | NP_077027.1 | NM_024122.5 |
| APOD | NP_001638.1 | NM_001647.4 |
| APOE | NP_001289617.1 | NM_001302688.2 |
| APOL1 | NP_003652.2 | NM_003661.4 |
| APOL2 | NP_112092.2 | NM_030882.4 |
| APOL3 | NP_663615.1 | NM_145640.2 |
| APOL4 | NP_085146.2 | NM_030643.4 |
| APOL5 | NP_085145.1 | NM_030642.1 |
| APOL6 | NP_085144.1 | NM_030641.4 |
| APOC1 | NP_001636.1 | NM_001645.5 |
| APOC2 | NP_000474.2 | NM_000483.5 |
| APOC4 | NP_001637.1 | NM_001646.3 |

The medicament of the present invention comprising an apolipoprotein as an active ingredient may be a medicament comprising a recombinant apolipoprotein as an active ingredient. In this case, the medicament of the present invention can be embodied in the form of a pharmaceutical preparation containing the recombinant apolipoprotein together with a pharmaceutically acceptable carrier. The recombinant apolipoprotein can be produced by appropriate known recombinant techniques, that is, by inserting a DNA encoding the apolipoprotein into an expression vector, introducing the resulting expression vector into an appropriate host cell to induce the expression of the apolipoprotein, and purifying the expressed apolipoprotein by a known method.

The medicament of the present invention comprising an apolipoprotein as an active ingredient may be a medicament comprising as an active ingredient a nucleic acid that is capable of expressing the apolipoprotein in cancer cells after administration of the medicament. In this case, the medicament of the present invention can be embodied in the form of a viral vector containing a DNA encoding the apolipoprotein, a mammalian expression vector containing a DNA encoding the apolipoprotein, an mRNA for the apolipoprotein, or a liposome containing the expression vector or the mRNA.

Interferon regulatory factor 7 (hereinafter referred to as "IRF7") is one of the transcription factors that bind to the expression regulatory region of the interferon (IFN)-β gene. The IRF7 used as an active ingredient in the medicament of the present invention may be IRF7 of any organism, but mammalian IRF7 is preferred. Mammals include, but are not limited to, humans, mice, rats, cattle, horses, pigs, dogs, and cats. Particularly preferably, human IRF7 is used. The human IRF7 gene is located at chromosome 11: 612555-615950 according to GRCh38/hg38. The nucleotide and amino acid sequences of the genes encoding human IRF7 can be obtained from known databases such as NCBI. The amino acid and nucleotide sequences listed in Table 2 are non-limiting examples.

**[Table 2]**

| Human IRF7 | Amino acid sequence (RefSeq ID) | Nucleotide sequence (RefSeq ID) |
|---|---|---|
| Isoform D | NP_004022.2 | NM_004031.4 |
| Isoform A | NP_001563.2 | NM_001572.5 |
| Isoform B | NP_004020.1 | NM_004029.4 |

The medicament of the present invention comprising IRF7 as an active ingredient may be a medicament comprising a recombinant IRF7 protein as an active ingredient. In this case, the medicament of the present invention can be embodied in the form of a pharmaceutical preparation containing the recombinant IRF7 protein together with a pharmaceutically acceptable carrier. The recombinant IRF7 protein can be produced by appropriate known recombinant techniques, that is, by inserting a DNA encoding IRF7 protein into an expression vector, introducing the resulting expression vector into an appropriate host cell to induce the expression of IRF7 protein, and purifying the expressed IRF7 protein by a known method.

The medicament of the present invention comprising IRF7 as an active ingredient may be a medicament comprising as an active ingredient a nucleic acid that is capable of expressing IRF7 protein in cancer cells after administration of the medicament. In this case, the medicament of the present invention can be embodied in the form of a viral vector containing a DNA encoding IRF7 protein, a mammalian expression vector containing a DNA encoding IRF7 protein, an mRNA for IRF7 protein, or a liposome containing the expression vector or the mRNA.

The medicament of the present invention may be embodied in the form of a single agent containing an apolipoprotein as the active ingredient, a single agent containing IRF7 as the active ingredient, or a combination agent containing an apolipoprotein and IRF7 as the active ingredients. Alternatively, a single agent containing an apolipoprotein as the active ingredient and a single agent containing IRF7 as the active ingredient may be used in combination.

The medicament of the present invention can be used in combination with a T-cell co-stimulator agonist. That is, the medicament of the present invention can be embodied in the form of a combination agent containing an apolipoprotein and a T-cell co-stimulator agonist as the active ingredients, a combination agent containing IRF7 and a T-cell co-stimulator agonist as the active ingredients, or a combination agent containing an apolipoprotein, IRF7, and a T-cell co-stimulator agonist as the active ingredients. Alternatively, a single agent containing an apolipoprotein as the active ingredient and a single agent containing a T-cell co-stimulator agonist may be used in combination; a single agent containing IRF7 as the active ingredient and a single agent containing a T-cell co-stimulator agonist may be used in combination; or a combination agent containing an apolipoprotein and IRF7 as the active ingredients and a single agent containing a T-cell co-stimulator agonist may be used in combination.

A combined use of the medicament of the present invention with a T-cell co-stimulator agonist can provide an anti-cancer effect on cancer tissue located at a distance from the target cancer tissue. For example, local administration of the medicament of the present invention and a T-cell co-stimulator agonist into a tumor results in not only growth inhibition of the treated tumor (target tumor), but also growth inhibition of a non-treated tumor located at a distance from the target tumor (non-target tumor) (see Examples).

Examples of the T-cell co-stimulator include OX40, 4-1BB, GITR, CD28, CD40, ICOS, HVEM, CD27, CD30, DR3, TNFR2, LTαβ, and LFA1. Examples of the T-cell co-stimulator agonist include agonistic antibodies to the T-cell co-stimulators listed above and ligands for the T-cell co-stimulators listed above. Preferred are agonists of OX40, 4-1BB, GITR, CD28, CD40, ICOS, and CD27. The agonistic antibody to T-cell co-stimulators can be a known antibody or a self-made antibody. Examples of the ligand for T-cell co-stimulators include OX40L/TNFSF4, 4-1-BB ligand/TNFSF9, GITR ligand/TNFSF18, CD80, CD86, ICOSLG, CD30L/TNFSF8, TL1A/TNFSF15, LIGHT/TNFSF14, TNFα, HVEM/TNFRSF14, and LTBR. The nucleotide and amino acid sequences of the genes encoding these ligands can be obtained from known databases such as NCBI. The amino acid and nucleotide sequences listed in Table 3 are non-limiting examples.

**[Table 3]**

| T-cell co-stimulator ligand | Amino acid sequence (RefSeq ID) | Nucleotide sequence (RefSeq ID) |
|---|---|---|
| OX40L/TNFSF4 | NP_001284491.1 | NM_001297562.2 |
| 4-1-BB ligand/TNFSF9 | NP_003802.1 | NM_003811.4 |
| GITR ligand/TNFSF18 | NP_005083.3 | NM_005092.4 |
| CD80 | NP_005182.1 | NM_005191.4 |
| CD86 | NP_787058.5 | NM_175862.5 |
| ICOSLG | NP_056074.1 | NM_015259.6 |
| CD30L/TNFSF8 | NP_001239219.1 | NM_001252290.1 |
| TL1A/TNFSF15 | NP_001191273.1 | NM_001204344.1 |
| LIGHT/TNFSF14 | NP_001191273.1 | NM_172014.3 |
| TNFα | NP_000585.2 | NM_000594.4 |
| HVEM/TNFRSF14 | NP_003811.2 | NM_003820.4 |
| LTBR | NP_002333.1 | NM_002342.3 |

The T-cell co-stimulator agonist can be administered in the form of a pharmaceutical preparation containing the antibody or a recombinant protein of the ligand together with a pharmaceutically acceptable carrier. Alternatively, the T-cell co-stimulator agonist can be administered in the form of a nucleic acid that is capable of expressing the ligand protein in cancer cells. In the latter case, the T-cell co-stimulator agonist can be administered in the form of a viral vector containing a DNA encoding the ligand protein, a mammalian expression vector containing a DNA encoding the ligand protein, an mRNA for the ligand protein, or a liposome containing the expression vector or the mRNA.

The medicament of the present invention may be a medicament comprising a combination of HVJ-E and a T-cell co-stimulator agonist in place of the apolipoprotein and IRF7. The HVJ-E can be prepared by inactivating HVJ. The HVJ inactivating method for preparing HVJ-E may be UV treatment or alkylation treatment. Such inactivation treatments cause the denaturation or fragmentation of viral genomic RNA inside the envelope, resulting in loss of its activity and viral replication capability. More specifically, the HVJ-E of the present invention can be prepared according to the methods described in JPA 2001-286282 (WO01/57204), JPA 2002-065278, WO03/014338, Kaneda et al. (Molecular Therapy 2002, 6, 2, 219-226), and other publications.

The HVJ-E may be administered via any appropriate route, preferably injected into the cancer tissue (tumor) or its surrounding area. The dose can be determined by a physician or healthcare professional according to the size of the cancer tissue (tumor), the patient's age, weight, condition, etc. When the tumor volume is less than 1,000 mm³ (e.g., about 200 mm³), the dose of HVJ-E per administration at a single tumor site can be 3 Hemagglutinin Unit (HAU) to 750,000 HAU, preferably 30 HAU to 300,000 HAU, more preferably 300 HAU to 200,000 HAU, and still more preferably 9,000 HAU to 100,000 HAU. Alternatively, the dose is preferably less than 100,000 HAU per kilogram of body weight.

The medicament of the present invention may be used in combination with a CD4-positive T-cell depleting antibody in addition to a T-cell co-stimulator agonist. Examples of the CD4-positive T-cell depleting antibody include humanized anti-CD4 antibodies "IT1208" (J Immunother Cancer. 2019 Jul 24;7(1):195. doi: 10.1186/s40425-019-0677-y.), "MAX.16H5" (Front Immunol. 2019 May 24;10:1035. doi: 10.3389/fimmu.2019.01035.), and "cM-T412" (J Clin Invest. 1997 May 1;99(9):2225-31. doi: 10.1172/JCI119396.). A combined use with the CD4-positive T-cell depleting antibody can provide an enhanced anti-cancer effect on non-target cancer tissue.

The cancer to be treated with the medicament of the present invention is not particularly limited, and examples include melanoma (malignant melanoma), Merkel cell carcinoma, lung cancer, mesothelioma, head and neck cancer, esophageal cancer, stomach cancer, liver cancer, biliary tract cancer, pancreatic cancer, colorectal cancer, prostate cancer, kidney cancer, bladder cancer, urothelial cancer, breast cancer, uterine cancer, ovarian cancer, brain tumor, thyroid cancer, angiosarcoma, rhabdomyosarcoma, leiomyosarcoma, fibrosarcoma, synovial sarcoma, liposarcoma, neuroendocrine tumor, lymphoma, leukemia, and myeloma.

The medicament of the present invention can be formulated in the usual manner into a dosage form containing, as an active ingredient, a gene product whose expression is increased in cancer cells by treatment of the cancer cells with HVJ-E. For example, the dosage form can be an oral preparation, and examples of the oral preparation include solid or liquid preparations, specifically tablets (including sugar-coated tablets and film-coated tablets), pills, granules, powders, capsules (including soft capsules), syrups, emulsions, suspensions, etc. These preparations can be produced by known methods and contain one or more carriers, diluents or excipients commonly used in the field of pharmaceutical formulation. For example, carriers or excipients used for tablets include lactose, starch, sucrose, and magnesium stearate. The dosage form can be a parenteral preparation, and examples include injections and suppositories. The injections include an intravenous injection, a subcutaneous injection, an intracutaneous injection, an intramuscular injection, an intravenous infusion, and an intraarticular injection. These injections are prepared according to known methods, for example, by dissolving, suspending or emulsifying the active ingredient in a sterile aqueous or oily liquid commonly used for injections. As an aqueous liquid for injections, for example, physiological saline, an isotonic solution containing glucose and an auxiliary substance, or the like can be used, optionally together with a suitable solubilizer such as alcohols (e.g., ethanol etc.), polyalcohols (e.g., propylene glycol, polyethylene glycol, etc.) and nonionic surfactants (e.g., polysorbate 80, HCO-50, etc.). As an oily liquid, for example, sesame oil, soybean oil, or the like can be used, optionally together with a solubilizer such as benzyl benzoate and benzyl alcohol. Suppositories for rectal administration are prepared by mixing the active ingredient with a base commonly used for suppositories. The pharmaceutical preparations as obtained in the above manner are safe and less toxic, and therefore can be administered orally or parenterally to, for example, humans and other mammals (e.g., rats, mice, rabbits, sheep, pigs, cows, cats, dogs, monkeys, etc.).

The medicament of the present invention comprising a recombinant protein or an antibody as the active ingredient is preferably administered in the form of an injection or an infusion containing the active ingredient and a pharmaceutically acceptable carrier via a parenteral route, for example, intravenously, intramuscularly, intracutaneously, intraperitoneally, subcutaneously, or locally. The antibody may be a polyclonal or monoclonal antibody. The antibody may be a whole antibody molecule or an antibody fragment (for example, Fab, F(ab')₂, Fab', Fv, scFv, etc.) capable of specifically binding to an antigen of interest. The antibody is preferably a chimeric human antibody or a humanized antibody.

The medicament of the present invention comprising a nucleic acid as the active ingredient can be administered in the form of a non-viral or viral vector. The medicament in the form of a non-viral vector can be administered using liposome-based techniques for introduction of nucleic acid molecules (the liposome method, the HVJ-liposome method, the cationic liposome method, the lipofection method, the lipofectamine method, etc.). Other techniques include microinjection techniques and gene gun techniques for introduction of nucleic acid molecules and a carrier (metal particles). For administration of the medicament in the form of a viral vector, a DNA constructed to express the protein of interest is introduced into a nontoxic DNA or RNA virus such as nontoxic strains of retrovirus, adenovirus, adeno-associated virus, herpesvirus, vaccinia virus, poxvirus, poliovirus, Sindbis virus, HVJ, or SV40, and target cancer cells are infected with the resulting recombinant virus to induce the expression of the active ingredient in the target cancer cells.

The amount of the active ingredient in the medicament of the present invention may be 0.001 to 50% by mass, and is preferably 0.01 to 10% by mass, more preferably 0.1 to 1% by mass. The dose of the medicament is determined by a physician or health care professional according to the type of cancer, the severity of the disease, and the patient's age, weight, sex, and medical history. For example, the daily dose for an average human subject weighing about 65 to 70 kg is preferably about 0.02 to 4000 mg, more preferably about 0.1 to 200 mg. The total daily dose may be given as a single dose or in divided doses.

### Immunostimulant

The present inventors found that administration of a T-cell co-stimulator agonist in combination with an apolipoprotein, IRF7, or HVJ-E resulted in increased T cell counts and T cell activation in cancer tissue located at a distance from the target cancer tissue (non-target cancer tissue). More specifically, the present inventors confirmed not only increased T-cell counts, but also increased expressions of interferon γ (hereinafter referred to as "IFNy") and granzyme A in non-target cancer tissue. Therefore, the present invention provides an immunostimulant comprising a combination of a T-cell co-stimulator agonist and at least one selected from the group consisting of an apolipoprotein, IRF7 and HVJ-E.

The immunostimulant of the present invention is preferably used as an immunostimulant for local administration. Local administration of the immunostimulant of the present invention into lesions can not only induce migration of T cells to local lesions, but also induce further migration of the T cells from the local lesions to distant lesions. Therefore, the immunostimulant of the present invention can be referred to as an immunostimulant for promoting migration of migrated T cells from local lesions to distant lesions, or an agent for promoting migration of migrated T cells from local lesions to distant lesions.

The immunostimulant of the present invention is suitable not only for use as a medicament for treating cancer, but also for use in the treatment of diseases in which lesions identical to target lesions occur at a distance from the target lesions. For example, the immunostimulant of the present invention is suitable for use in the treatment of infectious diseases in which distant foci of infection occur. Such infectious diseases include, for example, impetigo contagiosa, necrotizing fasciitis, cellulitis, osteomyelitis, meningitis, intractable skin ulcer (such as diabetic ulcer), tuberculosis, tinea, sexually transmitted diseases, herpes (herpes labialis, herpes genitalis), and herpes zoster.

The immunostimulant of the present invention can be produced and used in the same manner as the medicament of the present invention described above.

### Method for screening for an anti-cancer substance

The present invention provides a method for screening for an anti-cancer substance. The substance selected by the screening method of the present invention is useful as a candidate active ingredient of the medicament for treating cancer.

A first embodiment of the screening method of the present invention comprises the following steps of:
(1) bringing cancer cells into contact with a test substance,
(2) measuring the expression level of IRF7 in the cancer cells, and
(3) comparing the expression level of IRF7 measured in the previous step to that in the absence of contact with the test substance to identify a substance capable of increasing the expression level of IRF7.

In step (1), a test substance is brought into contact with cancer cells. The test substance to be screened is not particularly limited, and examples include nucleic acids, peptides, proteins, non-peptidic compounds, synthetic compounds, fermentation products, cell extracts, cell culture supernatants, plant extracts, mammalian tissue extracts, and plasma. The test substance may be a novel or known substance. The test substance may be in the form of a salt. The salt of the test substance may be a salt with a physiologically acceptable acid or base.

The cancer cells are not particularly limited and may be any cancer cells that are suitable for treatment with the medicament of the present invention. The cancer cells may be cell lines, primary cultured cells, or cells from biopsied tissues. The cancer cells may be genetically engineered cells. Preferable examples of the cancer cells include cancer cells transfected with a vector having a reporter gene fused downstream of a DNA fragment having IRF7 promoter activity, and cancer cells having a reporter gene knocked-in at the endogenous IRF7 gene locus.

The DNA fragment having IRF7 promoter activity can be, for example, a DNA fragment of -1123 to +575 of the IRF7 gene consisting of the nucleotide sequence shown in SEQ ID NO: 1 (J Biol Chem. 2005 Apr 1;280(13):12262-12270. doi: 10.1074/jbc.M404260200.). The reporter gene is not particularly limited and may be any commonly used reporter gene. Preferred is a stable reporter gene whose activity is easily measurable. Examples of the reporter gene include genes encoding luciferase, β-galactosidase, β-glucuronidase, chloramphenicol acetyltransferase, alkaline phosphatase, peroxidase, and green fluorescent protein (GFP).

The method for bringing the test substance into contact with the cancer cells is not particularly limited. For example, in the case where cultured cells or cultured tissues are used, the test substance is added to culture medium. The contact of the test substance with cells in the living body of a non-human organism can be achieved by systemic administration such as oral, intravenous or intraperitoneal administration or local administration to a target organ or tissue. In addition, it is preferable to provide a control group, that is, a group in the absence of contact with the test substance.

In step (2), the expression level of IRF7 in the cancer cells in contact with the test substance is measured. The expression level of IRF7 measured may be a protein level of IRF7 or an mRNA level of IRF7. The protein level of IRF7 can be measured by extracting proteins from the cancer cells by a known method and quantifying the amount of IRF7 protein by a known protein quantification method. Examples of the known protein quantification method include western blotting, EIA, ELISA, RIA, and a method using a protein assay reagent. The mRNA level of IRF7 can be measured by extracting RNAs by a known method and quantifying the amount of IRF7 mRNA by a known mRNA quantification method. Examples of the known mRNA quantification method include northern blotting, RT-PCR, quantitative RT-PCR, and RNase protection assay.

In the case of using the cancer cells transfected with a vector having a reporter gene fused downstream of a DNA fragment having IRF7 promoter activity, the expression level of IRF7 can be measured as the expression level of the reporter gene. The method for measuring the expression level of the reporter gene depends on the protein encoded by the reporter gene (reporter gene product). For example, when a reporter gene encoding luciferase is used, a cell lysate is prepared, luciferin as a substrate is added to the supernatant of the cell lysate, and the intensity of luminescence is measured using a commercially available detector to determine the expression level of the reporter gene. Other reporter genes are also available, and the amount of the corresponding reporter gene products can be measured by known methods.

In step (3), the expression level of IRF7 measured in step (2) is compared to that in the absence of contact with the test substance to identify a substance capable of increasing the expression level of IRF7. When the IRF7 protein or mRNA level or the reporter gene expression level in the cancer cells in contact with the test substance is increased as compared to that in the control group (not in contact with the test substance), the test substance can be identified as the desired substance. The degree of increase in the expression level of IRF7 is not particularly limited. For example, when the expression level of IRF7 in the cells in contact with the test substance is 1.5-fold, 1.6-fold, 1.7-fold, 1.8-fold, 1.9-fold, or 2-fold higher than that in the cells not in contact with the test substance, the test substance may be identified as the desired substance.

A second embodiment of the screening method of the present invention comprises the following steps of:
(1) bringing cancer cells into contact with a test substance,
(2) measuring the expression level of an apolipoprotein in the cancer cells, and
(3) comparing the expression level of the apolipoprotein measured in the previous step to that in the absence of contact with the test substance to identify a substance capable of increasing the expression level of the apolipoprotein.

The second embodiment can be performed in the same manner as the first embodiment, except that IRF7 in the first embodiment is changed to an apolipoprotein. Therefore, a detailed description of each step of the second embodiment will be omitted.

There are several types of apolipoproteins, but the screening method of the present invention comprises measuring the expression level of any one of them. The apolipoprotein whose expression level is to be measured can be selected from, for example, APOA1BP, APOOL, APOO, APOD, APOE, APOL1, APOL2, APOL3, APOL4, APOL6, APOC1, APOC2, and APOC4.

In the case of using cancer cells transfected with a reporter gene, the cancer cells include cancer cells transfected with a vector having a reporter gene fused downstream of a DNA fragment having apolipoprotein promoter activity of interest, and cancer cells having a reporter gene knocked-in at the endogenous apolipoprotein gene locus. The DNA fragment having APOD promoter activity can be, for example, a DNA fragment of -817 to +64 of the APOD gene consisting of the nucleotide sequence shown in SEQ ID NO: 2 (Mol Neurobiol. 2013 Dec;48(3):669-680. doi: 10.1007/s12035-013-8456-0.).

The present invention includes the following.
(a-1) A method for treating cancer, comprising administering to a mammal an effective amount of a gene product whose expression is increased in cancer cells by treatment of the cancer cells with hemagglutinating virus of Japan-envelope.
(a-2) A gene product for use in cancer treatment, wherein the expression of the gene product is increased in cancer cells by treatment of the cancer cells with hemagglutinating virus of Japan-envelope.
(a-3) Use of a gene product for production of a medicament for treating cancer, wherein the expression of the gene product is increased in cancer cells by treatment of the cancer cells with hemagglutinating virus of Japan-envelope.

In the aspects (a-1), (a-2) and (a-3), the active ingredient may be an apolipoprotein and/or interferon regulatory factor 7.

In the aspects (a-1), (a-2) and (a-3), a combined use with a T-cell co-stimulator agonist is also contemplated.
(b-1) A method for treating cancer, comprising administering to a mammal an effective amount of hemagglutinating virus of Japan-envelope and an effective amount of a T-cell co-stimulator agonist.
(b-2) A T-cell co-stimulator agonist for use in combination with hemagglutinating virus of Japan-envelope in cancer treatment.
(b-3) Hemagglutinating virus of Japan-envelope for use in combination with a T-cell co-stimulator agonist in cancer treatment.
(b-4) Use of a T-cell co-stimulator agonist in combination with hemagglutinating virus of Japan-envelope for production of a medicament for treating cancer.
(b-5) Use of hemagglutinating virus of Japan-envelope in combination with a T-cell co-stimulator agonist for production of a medicament for treating cancer.
(c-1) A method for stimulating immunity, comprising administering to a mammal an effective amount of at least one selected from the group consisting of an apolipoprotein, interferon regulatory factor 7, and hemagglutinating virus of Japan-envelope and an effective amount of a T-cell co-stimulator agonist.
(c-2) Use of a combination of a T-cell co-stimulator agonist and at least one selected from the group consisting of an apolipoprotein, interferon regulatory factor 7, and hemagglutinating virus of Japan-envelope for immunity stimulation.
(c-3) Use of a T-cell co-stimulator agonist in combination with at least one selected from the group consisting of an apolipoprotein, interferon regulatory factor 7, and hemagglutinating virus of Japan-envelope for production of an immunostimulant.
(c-4) Use of at least one selected from the group consisting of an apolipoprotein, interferon regulatory factor 7, and hemagglutinating virus of Japan-envelope in combination with a T-cell co-stimulator agonist for production of an immunostimulant.

### EXAMPLES

Hereinafter, the present invention will be described in detail by examples, but the present invention is not limited thereto.

### Experimental methods

### Tumor formation

The cancer cells used were B16F10 cells (mouse melanoma cell line), LL/2 cells (mouse lung adenocarcinoma cell line), MC38 cells (mouse colorectal cancer cell line), and CT26 cells (mouse colorectal cancer cell line). B16F10, LL/2, or MC38 cells were cultured in DMEM containing 10% FBS, 100 U/mL penicillin, and 0.1 mg/mL streptomycin. CT26 cells were cultured in RPMI containing 10% FBS, 100 U/mL penicillin, and 0.1 mg/mL streptomycin. The cells were cultured at 37°C in a 5% CO₂ atmosphere. The cells were confirmed to be mycoplasma-negative and then intracutaneously transplanted at 5 × 10⁵ cells/50 µL PBS/mouse. Tumor formation was confirmed 5 to 7 days after transplantation of the cancer cells, and the day when tumor formation was confirmed was designated as Day 0, the start of the experiment. Tumor size was measured every 2 days starting from Day 0. Tumor volume was determined by the formula: [tumor volume = major axis × minor axis × 1/2].

### HVJ-E preparation and injection

HVJ-E was prepared as described in Kaneda et al. (Molecular Therapy 2002, 6, 2, 219-226). UV-inactivated HVJ-E was centrifuged at 15,000 rpm at 4°C for 10 minutes, and the supernatant was discarded. The sediment was resuspended in PBS and used for injection. HVJ-E was injected intratumorally at 2000 HAU HVJ-E/50 µL a total of 3 times on Days 0, 2, and 4 (except for an experiment using PDXs).

### Cell preparation

Tet GFP, Tet GFP-Irf7, Tet GFP-Apod, Tet GFP-Apol9a, or Tet GFP-Apol9a-Apol9b-Apod DNA sequence was introduced into the ROSA region of B16F10 cells using the CRISPR/Cas9 system to prepare cells with Tet-inducible expression.

Irf7-deficient B16F10 cells and Apod-deficient B16F10 cells were prepared using the CRISPR/Cas9 system.

### Doxycycline treatment

Doxycycline hydrochloride (Dox) was dissolved in ultrapure water at 2 mg/mL. This solution was used as drinking water and given to mice from the day before the start of the experiment (Day -1) to Day 6. After Day 7, normal water was given to the mice. Separately, 200 µL of 2 mg/mL doxycycline solution was administered orally to mice using a sonde needle on consecutive days from the day before the start of the experiment (Day -1) to Day 6, and on Days 8, 10, and 12.

### Recombinant proteins

Apod, Apol9a, or Apol9b expression plasmid was introduced into Expi293F cells (Thermo Fisher) using an ExpiFectamine 293 Transfection Kit (Thermo Fisher). The culture supernatant was filtered through a Millex-GV filter (Low protein Binding, 0.22 µm, Merck Millipore) and then purified using an AKTA pure 25 M1 system (Cytiva). First, simple purification was performed using HiTrap DEAE FF (Cytiva, 17-5055-01) with start buffer (20 mM Tris pH 8.0) and elution buffer (20 mM Tris, 0.5 M NaCl pH 8.0). The fractions containing the protein of interest were then purified using HisTrap HP (Cytiva, 17-5247-01) with start buffer (20 mM sodium phosphate, 0.5 M NaCl, 20 mM imidazole pH 7.4) and elution buffer (20 mM sodium phosphate, 0.5 M NaCl, 500 mM imidazole pH 7.4). The purified protein solution was passed through a HiTrap Desalting column (Cytiva, 17-1408-01) for buffer exchange into PBS. A mixed solution of the three recombinant proteins (mass ratio = 1:1:1) was prepared and injected intratumorally at a dose of 35 µg/80 µL per injection.

### Antibodies

The antibodies used for intratumoral injection were Ultra-LEAF Purified anti-mouse CD134 (OX-40) clone: OX-86 (BioLegend 119431), an agonistic antibody to T-cell co-stimulator OX40, and InVivoMAb anti-mouse 4-1BB (CD137) clone LOB12.3 (BioXCell BE0169). Each antibody was injected intratumorally at 10 µg plus 2000 HAU HVJ-E/50 µL PBS.

The anti-CD4 antibody used for depleting CD4-positive T cells was InVivoMAb anti-mouse CD4 clone GK1.5 (BioXCell BE0003-1), and the anti-CD8 antibody for depleting CD8-positive T cells was InVivoMAb anti-mouse CD8α (2.43) clone LOB12.3 (BioXCell BE0061). These antibodies were injected intraperitoneally on Day -1, 0, 2, 4, and 6. The dose of the anti-CD4 antibody was 500 µg/200 µL per injection, and the dose of the anti-CD8 antibody was 100 µg/200 µL per injection.

### Reference Example 1: Examination of anti-tumor effect of HVJ-E in tumors formed in immunodeficient mice

### (1) Anti-tumor effect of HVJ-E in immunodeficient mice bearing tumor tissue grafts transplanted from melanoma patients

Tumor tissue was harvested from two consenting melanoma patients and transplanted into NOD-SCID mice (PDX: Patient-derived xenografts). The day when the tumor tissue was confirmed to be engrafted was designated as Day 0. Intratumoral injection of HVJ-E (2000 HAU HVJ-E/50 µL PBS) and measurement of tumor size (recording of tumor volume) were performed once every 2 days from Day 0 to Day 42 for the first case (PDX1) and from Day 0 to Day 38 for the second case (PDX2). The results are shown in Fig. 1. Fig. 1A shows the results obtained in NOD-SCID mice bearing a tumor tissue graft transplanted from a patient in the first case, and Fig. 1B shows the results obtained in NOD-SCID mice bearing a tumor tissue graft transplanted from a patient in the second case. HVJ-E showed an inhibitory effect on tumor growth in both PDXs.

### (2) Anti-tumor effect of HVJ-E in immunodeficient mice bearing transplanted murine melanoma cells

B16F10 cells were transplanted into SCID mice and NSG mice to form tumors in these mice. HVJ-E was injected intratumorally on Days 0, 2, and 4. Tumor volume was recorded every 2 days from Day 0 to Day 14. The results are shown in Fig. 2. Fig. 2A shows the results obtained in SCID mice, and Fig. 2B shows the results obtained in NSG mice. HVJ-E showed an inhibitory effect on tumor growth in the tumors formed in both immunodeficient mice.

### (3) Summary

SCID mice are immunodeficient mice lacking T and B cells. NOD-SCID mice are immunodeficient mice which lack T and B cells and have low NK cell activity. NSG mice are severely immunodeficient mice lacking T, B and NK cells. The above results revealed that at least T cells, B cells, and NK cells are not essential for the anti-tumor effect of HVJ-E on target tumors.

### Example 1: Analysis for genes whose expression is increased by HVJ-E treatment (1)

B16F10 cells were transplanted into GFP mice to form tumors. On Days 0, 2, and 4, 2000 HAU of HVJ-E/50 µL PBS was injected intratumorally to an HVJ-E treatment group, and 50 µL of PBS was injected intratumorally to a control group. On Day 5, tumors were harvested, and GFP-negative cells were collected by FACS. RNA extraction from the harvested cells and subsequent RNAseq were performed according to the usual method. The obtained sequence data was analyzed using STAR, StringTie, and DESeq2 in R. The genes whose expression was increased by HVJ-E treatment are shown in Fig. 3.

### Example 2: Examination of the effect of HVJ-E on tumors formed from Irf7-deficient mouse melanoma cells

The genes whose expression was increased by HVJ-E treatment (see Fig. 3) included genes encoding transcription factors (Irf7 and Batf2). In this example, the present inventors prepared mouse melanoma cells lacking these genes and examined the influence of these transcription factors on the anti-tumor effect of HVJ-E.

Irf7-deficient B16F10 cells were transplanted into C57BL/6N mice to form tumors. On Days 0, 2, and 4, HVJ-E was injected intratumorally to an HVJ-E treatment group, and PBS was injected intratumorally to a control group. Tumor volume was recorded every 2 days from Day 0 to Day 14. The results are shown in Fig. 4. Intratumoral injection of HVJ-E had no anti-tumor effect on tumors formed from Irf7-deficient B 16F 10 cells. In contrast, the anti-tumor effect of HVJ-E was still observed on tumors formed from Batf2-deficient B16F10 cells, although the data are not shown. These results revealed that the anti-tumor effect of HVJ-E requires an increased Irf7 expression in tumor tissue.

### Example 3: Examination of the effect of HVJ-E on tumors formed from Apod-deficient mouse melanoma cells

The present inventors examined which genes would be upregulated in an Irf7-dependent manner using Irf7-deficient cells and HVJ-non-responsive LL2 cells and found that the expression of Apod, Apol9a, and Apol9b genes was increased. In this example, the present inventors prepared mouse melanoma cells lacking Apod gene and examined the influence of this gene on the anti-tumor effect of HVJ-E.

Apod-deficient B16F10 cells were transplanted into C57BL/6N mice to form tumors. On Days 0, 2, and 4, HVJ-E was injected intratumorally to an HVJ-E treatment group, and PBS was injected intratumorally to a control group. Tumor volume was recorded every 2 days from Day 0 to Day 14. The results are shown in Fig. 5. Intratumoral injection of HVJ-E had no anti-tumor effect on tumors formed from mouse melanoma cells lacking Apod gene.

### Example 4: Analysis for genes whose expression is increased by HVJ-E treatment (2)

After the examination in Reference Example 1 (1), tumors were harvested from NOD-SCID mice bearing tumor tissue grafts transplanted from melanoma patients (PDX1 and PDX2), and mouse-derived cells were removed from the tumors. RNA extraction from the harvested human-derived cells and subsequent RNAseq were performed according to the usual method. The obtained sequence data was analyzed using STAR, StringTie, and DESeq2 in R.

The genes whose expression was increased by HVJ-E treatment are shown in Fig. 6. An increase in IRF7 expression was observed in human-derived tumors treated with HVJ-E, as in mouse-derived tumors. In addition, the expression of apolipoproteins APOA1BP, APOOL, APOO, APOD, APOE, APOL1, APOL2, APOL3, APOL4, APOL6, APOC1, APOC2, and APOC4 was also increased in both PDXs.

### Example 5: Examination of anti-tumor effect of Irf7

Based on the findings that Irf7 expression was increased in HVJ-E-treated tumors and that no anti-tumor effect of HVJ-E was observed in tumors formed from Irf7-deficient mouse melanoma cells, the present inventors examined whether induction of Irf7 expression in tumors would have an anti-tumor effect.

B16F10 cells transfected with Tet GFP-Irf7, which expresses both GFP and Irf7 in a doxycycline-inducible manner, were transplanted into C57BL/6N mice to form tumors (Tet GFP-Irf7 group). For control, B16F10 cells transfected with Tet-GFP, which expresses GFP alone in a doxycycline-inducible manner, were transplanted into C57BL/6N mice to form tumors (Tet GFP group). Each group was further divided into a doxycycline treatment group and a non-treatment group. The doxycycline treatment schedule was as described above. Tumor volume was recorded every 2 days from Day 0 to Day 14.

The results are shown in Fig. 7. Fig. 7A shows the results obtained in the Tet GFP group, and Fig. 7B shows the results obtained in the Tet GFP-Irf7 group. Induction of Irf7 expression in tumors resulted in a significant tumor growth inhibition.

### Example 6: Examination of anti-tumor effect of apolipoproteins (1)

Based on the findings that apolipoprotein expression was increased in HVJ-E-treated tumors and that no anti-tumor effect of HVJ-E was observed in tumors formed from Apod-deficient mouse melanoma cells, the present inventors examined whether induction of apolipoprotein expression in tumors would have an anti-tumor effect.

B16F10 cells transfected with Tet GFP-Apod, which expresses GFP and apolipoprotein D in a doxycycline-inducible manner, Tet GFP-Apol9a, which expresses GFP and apolipoprotein L9a in a doxycycline-inducible manner, or Tet GFP-Apol9a-Apol9b-Apod, which expresses GFP, apolipoprotein L9a, apolipoprotein L9b, and apolipoprotein D in a doxycycline-inducible manner, were transplanted into C57BL/6N mice to form tumors (Tet GFP-Apod group, Tet GFP-Apol9a group, and Tet GFP-Apol9a-Apol9b-Apod group). Each group was further divided into a doxycycline treatment group and a non-treatment group. The doxycycline treatment schedule was as described above. Tumor volume was recorded every 2 days from Day 0 to Day 14.

The results are shown in Fig. 8. Fig. 8A shows the results obtained in the Tet GFP-Apod group, Fig. 8B shows the results obtained in the Tet GFP-Apol9a group, and Fig. 8C shows the results obtained in the Tet GFP-Apol9a-Apol9b-Apod group. Induction of apolipoprotein expression in tumors by doxycycline treatment resulted in a significant tumor growth inhibition in each group.

### Example 7: Examination of anti-tumor effect of apolipoproteins (2)

B16F10 cells (wild type) were transplanted into C57BL/6N mice to form tumors. The mice were divided into two groups: an apolipoprotein group (Apolipoproteins) and a control group (Vehicle). A mixed solution (mass ratio = 1:1:1) of three recombinant apolipoproteins D, L9a, and L9b was injected intratumorally to the apolipoprotein group a total of 7 times on Days 0, 2, 4, 6, 8, 10 and 12 (35 µg/80 µL/shot). A vehicle (80 µL/shot) was injected intratumorally to the control group 7 times.

The results are shown in Fig. 9. Direct injection of apolipoproteins into tumors also resulted in a significant tumor growth inhibition. In this example, a mixture of apolipoproteins D, L9a, and L9b was injected, but apolipoprotein D, apolipoprotein L9a, or apolipoprotein L9b alone also exerts a sufficient anti-tumor effect, as demonstrated from the results of Example 6. In addition, these experimental results indicate that apolipoproteins (APOA1BP, APOOL, APOO, APOD, APOE, APOL1, APOL2, APOL3, APOL4, APOL6, APOC1, APOC2, and APOC4) whose expression was increased by HVJ-E treatment of human-derived tumors also exert anti-tumor effect, either alone or in combination.

### Example 8: Examination of systemic anti-tumor effect

### 8-1 Examination of the efficacy of HVJ-E in combination with OX40 agonistic antibody

### (1) Tumors formed from mouse melanoma B16F10 cells

B16F10 cells (wild type) were bilaterally transplanted into C57BL/6N mice to form tumors on both sides. The mice were divided into 4 groups: a group receiving HVJ-E plus agonistic antibody to T-cell co-stimulator OX40 (HVJE + OX40 group), a group receiving HVJ-E plus control IgG (HVJE + Ctrl IgG group), a group receiving OX40 agonistic antibody (OX40 group), and a group receiving control IgG (Ctrl IgG group). The doses and treatment schedule were as described above. Treatment was performed in the tumor on the left side. The volumes of target (left) and non-target (right) tumors were recorded every 2 days from Day 0 to Day 14 (see Fig. 10A).

The results are shown in Figs. 10B and 10C. Fig. 10B shows the results obtained in the target tumor lesion, and Fig. 10C shows the results obtained in the non-target tumor lesion. The HVJE + OX40 group and the HVJE + Ctrl IgG group showed a significant growth inhibition of target tumors. Meanwhile, the HVJE + OX40 group showed a significant growth inhibition of non-target tumors, whereas the HVJE + Ctrl IgG group did not show any growth inhibition of non-target tumors.

### (2) Tumors formed from mouse colorectal cancer CT26 cells

The experiment was performed in the same manner as in the above (1), except that B16F10 cells (wild type) were changed to CT26 cells (wild type) (see Fig. 11A). The results are shown in Figs. 11B and 11C. Fig. 11B shows the results obtained in the target tumor lesion, and Fig. 11C shows the results obtained in the non-target tumor lesion. The HVJE + OX40 group and the HVJE + Ctrl IgG group showed a significant growth inhibition of target tumors. Meanwhile, the HVJE + OX40 group showed a significant growth inhibition of non-target tumors, whereas the HVJE + Ctrl IgG group did not show any growth inhibition of non-target tumors.

### (3) Tumors formed from mouse colorectal cancer MC38 cells

The experiment was performed in the same manner as in the above (1), except that B16F10 cells (wild type) were changed to MC38 cells (wild type) (see Fig. 12A). The results are shown in Figs. 12B and 12C. Fig. 12B shows the results obtained in the target tumor lesion, and Fig. 12C shows the results obtained in the non-target tumor lesion. The HVJE + OX40 group and the HVJE + Ctrl IgG group showed a significant growth inhibition of target tumors. Meanwhile, the HVJE + OX40 group showed a significant growth inhibition of non-target tumors, whereas the HVJE + Ctrl IgG group did not show any growth inhibition of non-target tumors.

### 8-2 Examination of the efficacy of HVJ-E in combination with anti-OX40 antibody in immunodeficient mice (SCID mice)

The experiment was performed in the same manner as in the above 8-1 (1), except that SCID mice were used instead of C57BL/6N mice (see Fig. 13A). The results are shown in Figs. 13B and 13C. Fig. 13B shows the results obtained in the target tumor lesion, and Fig. 13C shows the results obtained in the non-target tumor lesion. The HVJE + OX40 group did not showed any growth inhibition of non-target tumors in SCID mice. The results demonstrate that the anti-tumor effect on non-target tumors is dependent on lymphocytes.

### 8-3 Examination of the efficacy of HVJ-E in combination with anti-PD-1 antibody

The experiment was performed in the same manner as in the above 8-1 (1), except that anti-PD-1 antibody, which is an immune checkpoint inhibitor, was used instead of OX40 agonistic antibody (see Fig. 14A). The results are shown in Figs. 14B and 14C. Fig. 14B shows the results obtained in the target tumor lesion, and Fig. 14C shows the results obtained in the non-target tumor lesion. The HVJE + PD-1 group did not show any growth inhibition of non-target tumors.

### 8-4 Examination of the efficacy of HVJ-E in combination with 4-1-BB agonistic antibody

The experiment was performed in the same manner as in the above 8-1 (1), except that an agonistic antibody to 4-1-BB, which is a T-cell co-stimulator like OX40, was used instead of OX40 agonistic antibody (see Fig. 15A). The results are shown in Figs. 15B and 15C. Fig. 15B shows the results obtained in the target tumor lesion, and Fig. 15C shows the results obtained in the non-target tumor lesion. The HVJE + 4-1-BB group showed a significant growth inhibition of non-target tumors.

These results demonstrate that stimulation with HVJ-E and T-cell co-stimulator can induce systemic anti-tumor effect.

### Example 9: Examination of tumor cell type specificity in systemic anti-tumor effect

In order to determine whether the systemic anti-tumor effect is specific to the type of tumor cells, mouse lung adenocarcinoma LL/2 cells were transplanted to form tumors instead of B16F10 cells. The formed tumors were used as non-target tumors. That is, the experiment was performed in the same manner as in the above 4-1, except that LL/2 cells were used to form non-target tumors instead of B16F10 cells (see Fig. 16A).

The results are shown in Figs. 16B and 16C. Fig. 16B shows the results obtained in the target tumor lesion, and Fig. 16C shows the results obtained in the non-target tumor lesion. A combined injection of HVJ-E and OX40 agonistic antibody to melanoma cell tumors (target tumors) resulted in no growth inhibition of lung adenocarcinoma cell tumors (non-target tumors). These results demonstrate that the systemic anti-tumor effect induced by a combined injection of HVJ-E and OX40 agonistic antibody is specific to the type of tumor cells.

### Example 10: Examination of the involvement of CD4-positive T cells and CD8-positive T cells in systemic anti-tumor effect

An experiment was conducted to clarify the role of CD4-positive T cells and CD8-positive T cells in the anti-tumor effect of HVJ-E and OX40 agonistic antibody on non-target tumors.

B16F10 cells were bilaterally transplanted into C57BL/6N mice to form tumors on both sides (see Fig. 17A). HVJ-E and OX40 agonistic antibody were injected into the tumor on the left side three times on Days 0, 2, and 4. In addition, an antibody for depleting CD4-positive T cells, an antibody for depleting CD8-positive T cells, or control IgG was injected intraperitoneally on Days -1, 0, 2, 4, and 6. The volumes of target (left) and non-target (right) tumors were recorded every 2 days from Day 0 to Day 14.

The results are shown in Figs. 17B and 17C. Fig. 17B shows the results obtained in the target tumor lesion, and Fig. 17C shows the results obtained in the non-target tumor lesion. Depletion of CD8-positive T cells resulted in a reduced anti-tumor effect on non-target tumors, whereas depletion of CD4-positive T cells resulted in an enhanced anti-tumor effect on non-target tumors. These results indicate that CD4-positive T cells regulate CD8-positive T cells to prevent excessive immune activation. In addition, the combined injection of HVJ-E, OX40 agonistic antibody, and CD4-positive T-cell depleting antibody was shown to induce a potent anti-tumor effect on non-target tumors.

### Example 11: Examination of the systemic anti-tumor effect of apolipoproteins in combination with OX40 agonistic antibody

Tet GFP-Apol9a-Apol9b-Apod-transfected B16F10 cells were transplanted into the left side of C57BL/6N mice, and wild-type B16F10 cells were transplanted into the right side to form tumors on both sides (see Fig. 18A). The doxycycline treatment schedule was as described above. OX40 agonistic antibody or control IgG was injected into the tumor on the left side three times on Days 0, 2, and 4. The volumes of target (left) and non-target (right) tumors were recorded every 2 days from Day 0 to Day 14. Body weights were also measured on Days 0, 4, 8, and 14.

The results are shown in Figs. 18B, 18C, and 18D. Fig. 18B shows the results of body weight measurement, Fig. 18C shows the results obtained in the target tumor lesion, and Fig. 18D shows the results obtained in the non-target tumor lesion. Induction of apolipoprotein expression in tumors by doxycycline treatment and intratumoral injection of OX40 agonistic antibody resulted in a potent tumor growth inhibition not only in target tumors but also in non-target tumors. No weight loss was observed throughout the monitoring period, indicating that no immune-related adverse events had occurred.

### Example 12: Examination of the systemic anti-tumor effect of Irf7 in combination with OX40 agonistic antibody

Tet GFP-Irf7-transfected B16F10 cells were transplanted into the left side of C57BL/6N mice, and wild-type B16F10 cells were transplanted into the right side to form tumors on both sides (see Fig. 19A). As control, Tet GFP-transfected B16F10 cells were transplanted into the left side of C57BL/6N mice, and wild-type B16F10 cells were transplanted into the right side to form tumors on both sides (see Fig. 20A). The doxycycline treatment schedule was as described above. OX40 agonistic antibody or control IgG was injected into the tumor on the left side three times on Days 0, 2, and 4. The volumes of target (left) and non-target (right) tumors were recorded every 2 days from Day 0 to Day 14.

The results obtained in mice bearing a target tumor lesion derived from Tet GFP-Irf7-transfected B16F10 cells are shown in Figs. 19B and 19C. The results obtained in mice bearing a target tumor lesion derived from Tet GFP-transfected B16F10 cells are shown in Figs. 20B and 20C. Figs. 19B and 20B show the results obtained in the target tumor lesion, and Figs. 19C and 20C show the results obtained in the non-target tumor lesion. Induction of Irf7 expression in tumors by doxycycline treatment and intratumoral injection of OX40 agonistic antibody resulted in tumor growth inhibition not only in target tumors but also in non-target tumors. In contrast, induction of GFP expression in tumors by doxycycline treatment and intratumoral injection of OX40 agonistic antibody resulted in no tumor growth inhibition in target tumors or non-target tumors. These results demonstrate that induction of Irf7 expression in tumors, which increases the expression of its downstream apolipoproteins, is also effective in inhibiting the growth of non-targeted tumors.

### Example 13: Examination of the systemic immune stimulatory effect of HVJ-E in combination with OX40 agonistic antibody

### (1) Evaluation of T cell counts in target and non-target tumors

B16F10 cells (wild type) were bilaterally transplanted into C57BL/6N mice to form tumors on both sides. The mice were divided into 4 groups: a group receiving HVJ-E plus agonistic antibody to T-cell co-stimulator OX40 (HVJE + OX40 group), a group receiving HVJ-E plus control IgG (HVJE + Ctrl IgG group), a group receiving PBS plus OX40 agonistic antibody (PBS + OX40 group), and a group receiving PBS plus control IgG (PBS + Ctrl IgG group). Doses are as described in the above "Experimental methods". Treatment was performed in the tumor on the left side (target tumor) three times on Days 0, 2, and 4. The target and non-target (right) tumors were harvested on Day 10 or Day 11. The numbers of CD4-positive T cells (CD45/CD3/CD4) and CD8-positive T cells (CD45/CD3/CD8) per 100,000 cells were measured by FACS (see Fig. 21A).

The results are shown in Figs. 21B and 21C. Fig. 21B shows the results obtained in the target tumor lesion, and Fig. 21C shows the results obtained in the non-target tumor lesion. A combined injection of HVJ-E and OX40 agonistic antibody resulted in significantly increased numbers of CD4-positive T cells and CD8-positive T cells not only in target tumors but also in non-target tumors. In contrast, injection of HVJ-E or the OX40 agonistic antibody alone resulted in no increase in the number of T cells.

### (2) Evaluation of T cell activation status in non-target cells

The numbers of IFNγ-positive cells and Ki67-positive cells in the Foxp3-negative CD4-positive T cell population and in the CD8-positive T cell population in non-target tumors in the HVJE + OX40 group and in HVJE + Ctrl IgG group were measured by FACS. Ki67 is a cell proliferation marker.

The results are shown in Fig. 22. The results are shown as the percentage (%) of IFNγ-positive cells or Ki67-positive cells in the Foxp3-negative CD4-positive T cell population and as the percentage (%) of IFNγ-positive cells or Ki67-positive cells in the CD8-positive T cell population. A combined injection of HVJ-E and OX40 agonistic antibody resulted in a significantly increased IFNγ expression as well as a significantly enhanced cell proliferation in T cells in non-target tumors.

### Example 14: Examination of the systemic immunostimulatory effect of apolipoproteins in combination with OX40 agonistic antibody

Tet GFP-Apol9a-Apol9b-Apod-transfected B16F10 cells were transplanted into the left side of C57BL/6N mice, and wild-type B16F10 cells were transplanted into the right side to form tumors on both sides (see Fig. 23A). The doxycycline treatment schedule was as described above. OX40 agonistic antibody or control IgG was injected into the tumor on the left side (target tumor) three times on Days 0, 2, and 4. The target and non-target (right) tumors were harvested on Day 14. The numbers of IFNγ-positive cells, Ki67-positive cells, granzyme A-positive cells, and granzyme B-positive cells in the CD4-positive T cell population and in the CD8-positive T cell population were measured by FACS. Granzymes A and B are enzymes that induce apoptosis of target cells.

The results are shown in Fig. 23B. The results are shown as the percentage (%) of cells positive for each marker in the CD4-positive T cell population and in the CD8-positive T cell population. The OX40 agonistic antibody treatment group showed a significantly increased IFNγ expression as well as a significantly enhanced cell proliferation in the CD4-positive T cell population and in the CD8-positive T cell population in non-target tumors compared to the control IgG treatment group. In addition, granzyme A expression was significantly increased in the CD8-positive T cell population in non-target tumors in the OX40 agonistic antibody treatment group compared to the control IgG treatment group.

### Example 15: Examination of the systemic anti-tumor and immunostimulatory effects of an apolipoprotein in combination with OX40 agonistic antibody

Tet GFP-Apod-transfected B16F10 cells were transplanted into the left side of C57BL/6N mice, and wild-type B16F10 cells were transplanted into the right side to form tumors on both sides (see Fig. 24A). The doxycycline treatment schedule was as described above. OX40 agonistic antibody or control IgG was injected into the tumor on the left side (target tumor) three times on Days 0, 2, and 4. The volumes of target (left) and non-target (right) tumors were recorded every 2 days from Day 0 to Day 14. The target and non-target (right) tumors were harvested on Day 14. The numbers of CD4-positive T cells (CD45/CD3/CD4) and CD8-positive T cells (CD45/CD3/CD8) per 100,000 cells were measured by FACS.

The results on anti-tumor effect are shown in Figs. 24B and 24C. Fig. 24B shows the results obtained in the target tumor lesion, and Fig. 24C shows the results obtained in the non-target tumor lesion. Induction of apolipoprotein expression in tumors by doxycycline treatment and intratumoral injection of OX40 agonistic antibody resulted in a potent tumor growth inhibition not only in target tumors but also in non-target tumors.

The results on the numbers of CD4-positive T cells and CD8-positive T cells are shown in Figs. 25A and 25B. Fig. 25A shows the results obtained in the target tumor lesion, and Fig. 25B shows the results obtained in the non-target tumor lesion. Induction of apolipoprotein expression in tumors by doxycycline treatment and intratumoral injection of OX40 agonistic antibody resulted in significantly increased numbers of CD4-positive T cells and CD8-positive T cells not only in target tumors but also in non-target tumors.

### Example 16: Examination of the systemic anti-tumor and immunostimulatory effects of an apolipoprotein in combination with OX40 agonistic antibody

B16F10 cells (wild type) were bilaterally transplanted into C57BL/6N mice to form tumors on both sides. The mice were divided into 4 groups: a group receiving 40 µg of purified Apod protein and OX40 agonistic antibody (Apod/OX40 group), a group receiving 40 µg of purified Apod protein and control IgG (Apod/Ctrl IgG group), a group receiving PBS and OX40 agonistic antibody (PBS/OX40 group), and a group receiving PBS and control IgG (PBS/Ctrl IgG group). Treatment was performed in the tumor on the left side (target tumor) five times on Days 0, 2, 4, 6, and 8. The volumes of target (left) and non-target (right) tumors were recorded every 2 days from Day 0 to Day 14. The target and non-target tumors were harvested on Day 14. The numbers of CD4-positive T cells (CD45/CD3/CD4) and CD8-positive T cells (CD45/CD3/CD8) per 100,000 cells were measured by FACS.

The results on anti-tumor effect are shown in Figs. 26A and 26B. Fig. 26A shows the results obtained in the target tumor lesion, and Fig. 26B shows the results obtained in the non-target tumor lesion. Intratumoral injection of the apolipoprotein and OX40 agonistic antibody resulted in a potent tumor growth inhibition not only in target tumors but also in non-target tumors.

The results on the numbers of CD4-positive T cells and CD8-positive T cells are shown in Fig. 27A and 27B. Fig. 27A shows the results obtained in the target tumor lesion, and Fig. 27B shows the results obtained in the non-target tumor lesion. Intratumoral injection of the apolipoprotein and OX40 agonistic antibody resulted in significantly increased numbers of CD4-positive T cells and CD8-positive T cells not only in target tumors but also in non-target tumors.

The present invention is not limited to particular embodiments and examples described above, and various modifications can be made within the scope of the appended claims. Other embodiments provided by appropriately combining technical means disclosed in separate embodiments of the present invention are also within the technical scope of the present invention. All the academic publications and patent literature cited in the description are incorporated herein by reference.

## Claims

1. A medicament for treating cancer comprising, as an active ingredient, a gene product whose expression is increased in cancer cells by treatment of the cancer cells with hemagglutinating virus of Japan-envelope.

2. The medicament for treating cancer according to claim 1, wherein the active ingredient is an apolipoprotein and/or interferon regulatory factor 7.

3. The medicament for treating cancer according to claim 1 or 2, wherein the medicament is used in combination with a T-cell co-stimulator agonist.

4. A medicament for treating cancer comprising a combination of hemagglutinating virus of Japan-envelope and a T-cell co-stimulator agonist.

5. The medicament for treating cancer according to claim 3 or 4, wherein the medicament is used in combination with a CD4-positive T-cell depleting antibody.

6. An immunostimulant comprising a combination of a T-cell co-stimulator agonist and at least one selected from the group consisting of an apolipoprotein, interferon regulatory factor 7, and hemagglutinating virus of Japan-envelope.

7. The immunostimulant according to claim 6, wherein the immunostimulant is for local administration.

8. The immunostimulant according to claim 6 or 7, wherein the immunostimulant is used in combination with a CD4-positive T-cell depleting antibody.

9. A method for screening for an anti-cancer substance, comprising the steps of:
(1) bringing cancer cells into contact with a test substance,
(2) measuring the expression level of interferon regulatory factor 7 (IRF7) in the cancer cells, and
(3) comparing the expression level of IRF7 measured in the previous step to that in the absence of contact with the test substance to identify a substance capable of increasing the expression level of IRF7.

10. A method for screening for an anti-cancer substance, comprising the steps of:
(1) bringing cancer cells into contact with a test substance,
(2) measuring the expression level of an apolipoprotein in the cancer cells, and
(3) comparing the expression level of the apolipoprotein measured in the previous step to that in the absence of contact with the test substance to identify a substance capable of increasing the expression level of the apolipoprotein.
